# EUROPEAN PATENT APPLICATION

(11) **EP 4 559 600 A1**
(43) Date of publication of application: **28.05.2025**
(21) Application number: 23842982.3
(22) Date of filing: 18.07.2023
(51) Int. Cl.: B22F 10/38, A61F 2/30, B22F 10/28, B28B 1/30, B33Y 10/00, B33Y 80/00

(54) **INORGANIC STRUCTURE AND METHOD FOR PRODUCING INORGANIC STRUCTURE**

(30) Priority: 19.07.2022 JP 2022115147
(71) Applicant: OSAKA UNIVERSITY, Suita-shi Osaka 565-0871 (JP)
(72) Inventor: NAKANO, Takayoshi, Suita-shi, Osaka 565-0871 (JP); ISHIMOTO, Takuya, Suita-shi, Osaka 565-0871 (JP); YASUDA, Hiroyuki, Suita-shi, Osaka 565-0871 (JP); ZHAO, Yan, Suita-shi, Osaka 565-0871 (JP)
(74) Representative: Zimmermann & Partner Patentanwälte mbB
(86) International application number: PCT/JP2023/026321
(87) International publication number: WO 2024/019059

(57) **Abstract**

An inorganic structure having mechanical properties that differ depending on the region in the inorganic structure, and a method for manufacturing the inorganic structure are provided. An inorganic structure (1) of the present embodiment includes a plurality of solidified portions (SA) composed of an inorganic material. The plurality of solidified portions (SA) include a first solidified portion (SA1) having a first crystallographic direction (CO1) preferentially oriented in a predetermined direction, and a second solidified portion (SA2) having a second crystallographic direction (CO2) that is a different orientation from the first crystallographic direction (CO1).

## Description

### TECHNICAL FIELD

The present disclosure relates to an inorganic structure that is made of an inorganic material, and a method for manufacturing the inorganic structure, and more particularly relates to an inorganic structure formed by an additive manufacturing process of AM (additive manufacturing), and a method for manufacturing the inorganic structure.

### BACKGROUND ART

Inorganic structures made of ceramic material or metal are sometimes used in medical implants typified by artificial joints and bone plates, mobile bodies typified by automobiles, aircraft, boats and ships, and the like, and industrial equipment such as plant equipment and manufacturing equipment. Such inorganic structures are required to have mechanical properties such as a certain Young's modulus or strength or shock absorption properties according to the application.

For example, when applying an inorganic structure to a medical implant, in some cases, the inorganic structure is required to have a Young's modulus equal to the Young's modulus of a biological bone. However, the Young's modulus of a solid inorganic structure is higher than the Young's modulus of a biological bone. Therefore, when a solid inorganic structure is implanted into a living body as an implant, a biological bone may be destroyed at the interface between the biological bone and the implant due to a difference in the Young's modulus between the biological bone and the implant, or normal stress may not be applied to the biological bone. Such a phenomenon is referred to as "stress shielding". In order to suppress stress shielding, it is necessary to adjust the Young's modulus of the inorganic structure to be equal to the Young's modulus of biological bone.

Further, when applying an inorganic structure to a mobile body, the mechanical properties required of the inorganic structure differ depending on the region within the mobile body at which the inorganic structure is to be applied. For example, in some cases, an inorganic structure will be required to have a high Young's modulus in a certain region, while in another region the inorganic structure will be required to have a low Young's modulus. Conventional inorganic structures are made using solid metal or the like as a raw material, and are formed into an inorganic structure of a predetermined shape by performing hot working, cold working, or the like on the metal or the like that is the raw material. However, in the case of an inorganic structure formed by a conventional manufacturing process, it is difficult to impart different mechanical properties that depend on the region in the inorganic structure.

Therefore, the present inventors have proposed inorganic structures whose mechanical properties are adjustable in Japanese Patent Application Publication No. 2011-136083 (Patent Literature 1) and Japanese Patent Application Publication No. 2013-94390 (Patent Literature 2).

The structures disclosed in Patent Literature 1 and Patent Literature 2 include a solidified portion that is formed by dissolving inorganic powder particles, and a sintered portion that is formed by sintering inorganic powder particles and is connected to the solidified portion. Because the sintered portion has spaces therein, the density is low in comparison to a solid material. Therefore, in the inorganic structure disclosed in Patent Literature 1, the modulus of elasticity can be lowered as compared to a solid inorganic structure. This kind of inorganic structure can be formed, for example, by an additive manufacturing process of AM (additive manufacturing).

### CITATION LIST

### PATENT LITERATURE

Patent Literature 1: Japanese Patent Application Publication No. 2011-136083
Patent Literature 2: Japanese Patent Application Publication No. 2013-94390

### SUMMARY OF INVENTION

### TECHNICAL PROBLEM

In the inorganic structures of each of the aforementioned Patent Literature 1 and Patent Literature 2, the density at each region in the inorganic structure is adjusted so that the inorganic structure has different mechanical properties depending on the region in the inorganic structure. However, other means may also be used to impart mechanical properties that differ depending on the region.

An objective of the present disclosure is to provide an inorganic structure having mechanical properties that differ depending on the region in the inorganic structure, and a method for manufacturing the inorganic structure.

### SOLUTION TO PROBLEM

An inorganic structure according to the present disclosure includes a plurality of solidified portions composed of an inorganic material. A plurality of solidified portions include a first solidified portion and a second solidified portion. The first solidified portion has a crystallographic orientation in which a first crystallographic direction is preferentially oriented in a predetermined direction. The second solidified portion has a crystallographic orientation in which a second crystallographic direction that is a different direction from the first crystallographic direction is preferentially oriented in the predetermined direction.

In the inorganic structure according to the present disclosure, the first and second solidified portions have different preferential crystallographic orientations from each other. Therefore, the first and second solidified portions have different mechanical properties from each other. Therefore, an inorganic structure of the present embodiment has mechanical properties that differ depending on the region in the inorganic structure.

Preferably, the solidified portion includes a plurality of sub-solidified areas that are each surrounded by a plurality of molten pool interfaces. The sub-solidified area includes a plurality of cells, and a plurality of cell interfaces which are interfaces between the cells that are adjacent to each other.

**In** this case, mechanical properties such as the strength and Young's modulus are increased by the cells and cell interfaces.

Preferably, the first solidified portion has a different shape from the second solidified portion.

In this case, because of a difference in crystallographic orientation and a difference in shape, the inorganic structure has mechanical properties that differ depending on the region in the inorganic structure.

Preferably, the sub-solidified area includes a plurality of cell areas that are each composed of a plurality of the cells, and a lamellar interface that is an interface between the cell areas that are adjacent. At the lamellar interface, one of the cell areas that are adjacent to each other has a different preferential crystallographic orientation from a crystallographic orientation of the other of the cell areas.

In this case, at the lamellar interface, solute segregation occurs at the atomic level. Mechanical properties such as the strength and Young's modulus are increased by the solute segregation.

Preferably, the sub-solidified area includes a plurality of cell areas that are each composed of a plurality of the cells, and an association interface that is an interface between the cell areas that are adjacent. At the association interface, one of the cell areas that are adjacent to each other has the same preferential crystallographic orientation as a crystallographic orientation of the other of the cell areas.

In this case, at the association interface, solute segregation occurs at the atomic level. Mechanical properties such as the strength and Young's modulus are increased by the solute segregation.

Preferably, the inorganic structure further includes a sintered portion that is a sintered compact of an inorganic material.

In this case, because of a difference in crystallographic orientation and a difference in form (solidified portion or sintered portion), the inorganic structure has mechanical properties that differ depending on the region in the inorganic structure.

Preferably, a space is formed between the solidified portions that are adjacent.

In this case, because of a difference in crystallographic orientation and a difference in shape, the inorganic structure has mechanical properties that differ depending on the region in the inorganic structure.

Preferably, in the first solidified portion, any one of <001>, <011>, and <111> is preferentially oriented in a build direction. In the second solidified portion, any one of <001>, <011>, and <111> which is different from the crystallographic orientation of the first solidified portion, is preferentially oriented in a build direction.

In this case, the first and second solidified portions have different preferential crystallographic orientations from each other. Therefore, the inorganic structure has mechanical properties that differ depending on the region in the inorganic structure.

Preferably, the solidified portion is composed of a single crystal, a single-crystal-like structure, or a polycrystal.

A method for manufacturing an inorganic structure according to the present disclosure includes a design step and a formation step. In the design step, a scanning method for scanning a beam for forming the inorganic structure to be manufactured is determined. In the formation step, the beam is scanned based on the scanning method for scanning the beam determined in the design step, and inorganic powder particles that serve as a raw material of the inorganic structure are melted to form the inorganic structure. The design step includes a step of dividing the inorganic structure to be manufactured into a plurality of solidified layers in a build direction, a step of partitioning the respective solidified layers into a plurality of solidified areas as viewed from the build direction, an orientation determination step, and a scanning method determination step. In the orientation determination step, an orientation of a crystallographic direction of the respective solidified areas is determined based on mechanical properties required for the respective solidified areas. In the scanning method determination step, a scanning method for scanning a beam in the respective solidified areas is determined based on a determined orientation of a crystallographic direction. In the formation step, a layer formation step and a melting step are repeated alternately to build a plurality of the solidified layers to form an inorganic structure having the configuration described above. In the layer formation step, inorganic powder particles are supplied onto a base to form an inorganic powder layer. In the melting step, the beam is scanned based on a scanning method for scanning a beam determined in the scanning method determination step to melt the inorganic powder layer and form the solidified layer that is composed of a plurality of the solidified areas having the crystallographic orientation determined in the orientation determination step.

In the manufacturing method according to the present invention, an inorganic structure of the present disclosure that has the configuration described above can be manufactured.

Preferably, in the orientation determination step, a crystallographic orientation in the build direction of the respective solidified areas is determined as being any one crystallographic direction among <001>, <011>, and <111>. In the scanning method determination step, in the solidified area for which a <001> crystallographic orientation is determined, a scanning direction of the beam when forming the solidified layer of a j-th layer (j is a natural number) is adjusted so as to be orthogonal to a scanning direction of the beam when forming the solidified layer of a j-1th layer that is a layer underneath the j-th layer. In the solidified area for which a <011> crystallographic orientation is determined, a scanning direction of the beam when forming the solidified layer of a j-th layer is adjusted so as to be parallel to a scanning direction of the beam when forming the solidified layer of the j-1th layer. In the solidified area for which a <111> crystallographic orientation is determined, a scanning direction of the beam when forming the solidified layer of a j-th layer is adjusted so as to intersect at 120° with respect to a scanning direction of the beam when forming the solidified layer of the j-1th layer.

In this case, the crystallographic direction in the build direction of the solidified portion can be preferentially oriented to any one of <001>, <011>, and <111>.

Preferably, in the scanning method determination step, the scanning method for scanning the beam includes information relating to a scanning path of the beam in the solidified area of a size that serves as a standard. In the melting step, in a case where the solidified area to be melted is smaller than the solidified area of the size that serves as a standard, the beam is irradiated on a portion of a scanning path of the beam, which overlaps with the solidified area to be melted, and irradiation of a beam is stopped at a portion on the scanning path of the beam, which does not overlap with the solidified area to be melted.

In this case, even when a solidified area is a size that is smaller than a solidified area of a size that serves as a standard, the solidified area will be melted with a thermal history that is equal to the thermal history of the solidified area of the size that serves as a standard. As a result, even in a solidified area with a small size, a solidified structure that is the same as the solidified structure of a solidified area of a size that serves as a standard is obtained.

### BRIEF DESCRIPTION OF DRAWINGS

[FIG. 1] FIG. 1 is a perspective view illustrating an example of an inorganic structure according to the present embodiment.
[FIG. 2] FIG. 2 is a perspective view illustrating an inorganic structure according to a first embodiment.
[FIG. 3] FIG. 3 is a perspective view in which respective solidified portions in FIG. 2 are separated for the purpose of description.
[FIG. 4] FIG. 4 is a graph illustrating the relation between crystallographic orientations and Young's modulus in a case where the inorganic material is a titanium alloy and solidified portions are single crystals.
[FIG. 5] FIG. 5 is a schematic diagram illustrating the microstructure of a solidified portion of the inorganic structure illustrated in FIG. 2.
[FIG. 6] FIG. 6 is a diagram illustrating the relation between the presence or absence of cell interfaces in a solidified portion and the hardness (GPa) measured by nano-indentation.
[FIG. 7] FIG. 7 is a perspective view of an inorganic structure according to a second embodiment.
[FIG. 8A] FIG. 8A is a photographic image illustrating the microstructure of a sub-solidified area that is different from FIG. 5.
[FIG. 8B] FIG. 8B is a schematic diagram of the photographic image in FIG. 8A.
[FIG. 9A] FIG. 9A is a photographic image showing the microstructure of a sub-solidified area that is different from FIG. 5 and from FIG. 8A and FIG. 8B.
[FIG. 9B] FIG. 9B is a schematic diagram of the photographic image in FIG. 9A.
[FIG. 9C] FIG. 9C is a perspective view of the sub-solidified area shown in FIG. 9A.
[FIG. 10A] FIG. 10A is a schematic diagram of the microstructure of a sub-solidified area in a case where a crystallographic direction orients in a build direction Z is <001>.
[FIG. 10B] FIG. 10B is a perspective view of the sub-solidified area illustrated in FIG. 10A.
[FIG. 11A] FIG. 11A is a perspective view of a sub-solidified area in a case where a crystallographic direction orients in a build direction Z is <111>.
[FIG. 11B] FIG. 11B is a schematic diagram in which an inorganic structure 1 in which a crystallographic direction orients in a build direction Z is <111> is seen from the build direction Z.
[FIG. 12] FIG. 12 is a perspective view of an inorganic structure of an embodiment that is different from FIG. 2.
[FIG. 13] FIG. 13 is a perspective view of an inorganic structure of an embodiment that is different from FIG. 12.
[FIG. 14] FIG. 14 is a perspective view of an inorganic structure of a sixth embodiment.
[FIG. 15] FIG. 15 is a diagram illustrating the relation between various inorganic structures having different shapes and crystallographic orientations, and Young's modulus in the X direction, Y direction, and Z direction.
[FIG. 16] FIG. 16 is a schematic diagram of an additive manufacturing device for manufacturing the inorganic structure of the present embodiment.
[FIG. 17] FIG. 17 is a flowchart of a method for manufacturing the inorganic structure of the present embodiment.
[FIG. 18] FIG. 18 is a flowchart of a design step in the flowchart in FIG. 17.
[FIG. 19] FIG. 19 is a schematic diagram for describing solidified layers into which the inorganic structure is divided in the build direction.
[FIG. 20] FIG. 20 is a schematic diagram illustrating a state in which a solidified layer illustrated in FIG. 19 is partitioned into a plurality of solidified areas DA.
[FIG. 21] FIG. 21 is a view illustrating a scanning method (scanning paths) for a beam in a case where the crystallographic orientation adopted in a solidified area is <001>.
[FIG. 22] FIG. 22 is a view illustrating a scanning method (scanning paths) for a beam in a case where the crystallographic orientation adopted in a solidified area is <011>.
[FIG. 23] FIG. 23 is a view illustrating a scanning method (scanning paths) for a beam in a case where the crystallographic orientation adopted in a solidified area is <111>.
[FIG. 24] FIG. 24 is a flowchart of a formation step in the flowchart in FIG. 17.
[FIG. 25] FIG. 25 is a schematic diagram illustrating one step in the formation step in FIG. 24.
[FIG. 26] FIG. 26 is a schematic diagram illustrating a step that follows the step in FIG. 25.
[FIG. 27] FIG. 27 is a schematic diagram illustrating a step that follows the step in FIG. 26.
[FIG. 28] FIG. 28 is a schematic diagram illustrating a step that follows the step in FIG. 27.
[FIG. 29] FIG. 29 is a schematic diagram illustrating a step that follows the step in FIG. 28.
[FIG. 30] FIG. 30 is a schematic diagram illustrating a step that follows the step in FIG. 29.
[FIG. 31A] FIG. 31A is a diagram illustrating a scanning path of a beam within a solidified area of a size that serves as a standard.
[FIG. 31B] FIG. 31B is a schematic diagram for describing a scanning path of a beam in a solidified area that is smaller in size than a solidified area of a size that serves as a standard.
[FIG. 32] FIG. 32 a view showing pole figures for illustrating an effect obtained by providing an off time period.
[FIG. 33] FIG. 33 illustrates plan views of inorganic structures used in a test for obtaining FIG. 32.

### DESCRIPTION OF EMBODIMENTS

### [Overview of inorganic structure of present embodiment]

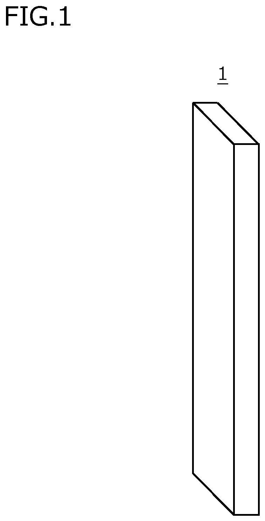

FIG. 1 is a perspective view illustrating an example of an inorganic structure 1 according to the present embodiment. The inorganic structure 1 is, for example, a medical implant. The inorganic structure 1 can also be used for applications other than medical implants. In the following description, as one example of the inorganic structure 1, a case where the inorganic structure 1 is applied to a medical implant is described.

The bone density of a biological bone varies depending on the region of the biological bone. Specifically, a biological bone includes cortical bone that covers the outer surface, and cancellous bone that is formed on the inside. In a biological bone, the density of cancellous bone increases at a region where high stress is applied. On the other hand, in a biological bone, the density of cancellous bone is lower at a region where only low stress is applied. Thus, the mechanical properties of a biological bone differ depending on the region in the biological bone.

In the case of utilizing the inorganic structure 1 as a substitute for a biological bone, it is preferable that the mechanical properties of the inorganic structure 1 are equal to the mechanical properties of the biological bone that the inorganic structure 1 is being substituted for. In other words, the inorganic structure 1 is required to have mechanical properties that differ depending on the region in the inorganic structure 1.

In each of Patent Literature 1 and Patent Literature 2, a solid solidified portion which is formed by melting and solidifying metal powder particles, and a sintered portion which is formed by sintering metal powder particles are prepared as elements that constitute the structure. The strength of the solidified portion is higher than the strength of the sintered portion. Therefore, in the structure, solidified portions are arranged at regions where high stress is applied, and sintered portions are arranged at regions where low stress is applied. Thus, in Patent Literature 1 and Patent Literature 2, regions (solidified portions and sintered portions) that have different physical structures (densities) are provided in the structure. By this means, the structure has mechanical properties that differ depending on the region.

In this connection, an inorganic material has a crystalline structure, and has a crystallographic orientation. For example, in the case of an inorganic material that has a cubic crystalline structure, the mechanical properties differ between the <001> crystallographic direction, the <011> crystallographic direction, and the <111> crystallographic direction. Therefore, in a case where the inorganic structure 1 is composed of a plurality of solidified portions in which the preferential crystallographic orientations in a predetermined direction are different from each other, the inorganic structure 1 can have mechanical properties that differ depending on the region in the inorganic structure 1.

The inorganic structure of the present embodiment was completed based on the technical idea described above. Hereunder, the structure of the present embodiment is described.

### [First embodiment]

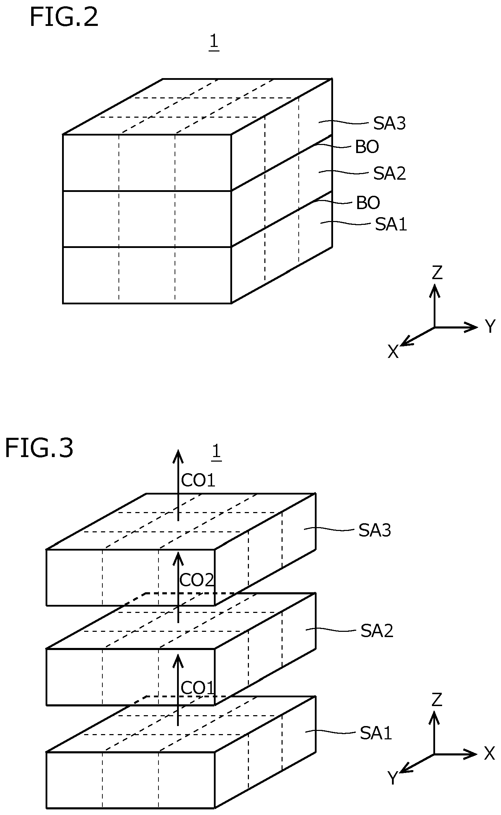

FIG. 2 is a perspective view of an inorganic structure according to a first embodiment. An inorganic structure 1 includes a plurality of solidified portions SA (SA1 to SA3). Note that, a boundary BO is formed between solidified portions SA that are adjacent. Each solidified portion SA is composed of an inorganic material. Specifically, each solidified portion SA is formed by melting and solidifying a plurality of inorganic powder particles. The inorganic powder particles are powder particles composed of an inorganic substance. The inorganic powder particles are composed of, for example, a metal, an intermetallic compound, a ceramic material, or the like. The metal is a pure metal or an alloy. Preferably, the inorganic powder particles are metal powder particles.

In FIG. 2, the inorganic structure 1 includes three solidified portions SA (SA1 to SA3). However, the inorganic structure 1 may include two solidified portions SA, or may include four or more solidified portions SA. The plurality of solidified portions SA of the inorganic structure 1 are integrally formed by an additive manufacturing process of AM (additive manufacturing) that is described later.

In the following, the description is continued based on the assumption that the inorganic structure 1 is arranged in an orthogonal coordinate system (X axis, Y axis, Z axis) that is illustrated in FIG. 2. Here, the Z direction means the build direction in an additive manufacturing process that is described later. In the following description, the Z direction, which is the build direction, is also referred to as " build direction Z". It is possible to identify the build direction Z from the external appearance of the inorganic structure 1. It is also possible to identify the build direction Z by etching a cross section of the inorganic structure 1.

FIG. 3 is a perspective view in which the respective solidified portions SA in FIG. 2 are separated for the purpose of description. Referring to FIG. 2 and FIG. 3, the solidified portion SA1 and the solidified portion SA2 are preferentially oriented to mutually different crystallographic directions. For example, in the solidified portion SA1, a crystallographic direction CO1 is preferentially oriented in the build direction Z. In the solidified portion SA2, a crystallographic direction CO2 that is a different crystallographic direction from the crystallographic direction CO1 is preferentially oriented in the build direction Z. Here, the term "preferentially oriented" means a state in which the orientation ratio of a specific crystallographic orientation in a specific direction of the inorganic structure 1 is higher than an orientation ratio measured in a case where the orientation is disordered.

Note that, in FIG. 3, in the solidified portion SA3, the same crystallographic direction CO1 as the solidified portion SA1 is preferentially oriented in the build direction Z.

Referring to FIG. 2, the boundary BO is formed between adjacent solidified portions SA. In other words, solidified portions SA that are adjacent are integrally formed, and the boundary BO exists between the adjacent solidified portions SA.

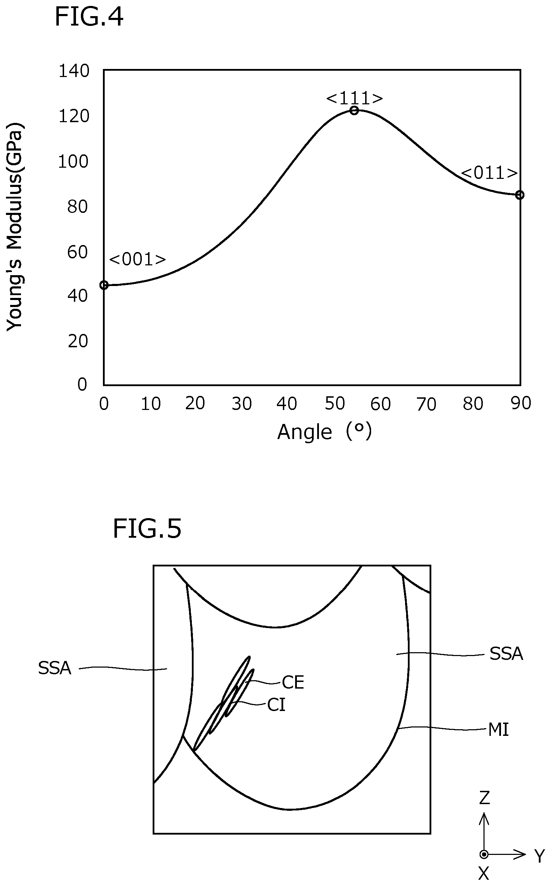

In the inorganic structure 1 having the configuration described above, by arranging a plurality of solidified portions that are preferentially oriented to different crystallographic directions, the inorganic structure 1 can have different mechanical properties depending on the region in the inorganic structure 1. For example, let us assume that in FIG. 2 and FIG. 3 the crystallographic direction CO1 is <111> and the crystallographic direction CO2 is <001>. FIG. 4 is a graph illustrating the relation between crystallographic directions and Young's modulus in a case where the solidified portions SA are single crystals, that is a case where the inorganic material is a titanium alloy. Referring to FIG. 4, in a titanium alloy in which the crystalline structure is a cubic crystalline structure, the Young's modulus at the <111> direction is the highest and the Young's modulus at the <001> direction is the lowest.

As illustrated in FIG. 2 and FIG. 3, in the inorganic structure 1, the preferentially oriented crystallographic direction of the solidified portion SA2 provided at the center portion in the build direction Z is to <001>. Further, the preferentially oriented crystallographic direction of the solidified portions SA1 and SA3 is <111>. Therefore, in the build direction Z, the Young's modulus of the solidified portions SA1 and SA3 is higher than the Young's modulus of the solidified portion SA2. Therefore, the inorganic structure 1 has mechanical properties that differ depending on the region in the inorganic structure 1.

For example, in a case where the inorganic structure 1 is a medical implant, and the solidified portions SA1 and SA3 of the inorganic structure 1 are to be fixed to a biological bone with bolts or the like, the solidified portions SA1 and SA3 are required to have a high Young's modulus in the build direction Z, while on the other hand, the solidified portion SA2 is required to have a low Young's modulus that is close to the Young's modulus of the biological bone. In this case, in the inorganic structure 1, as described above, the Young's modulus in the build direction Z of the solidified portions SA1 and SA3 can be made lower than the Young's modulus in the build direction Z of the solidified portion SA2. Thus, in the inorganic structure 1, by including a plurality of solidified portions that have different preferential orientation, mechanical properties that differ depending on the region in the inorganic structure 1 can be obtained.

The solidified portions SA may be composed of a pure metal or may be composed of an alloy. The solidified portions SA may be composed of an inorganic material other than a metal, such as a ceramic material. When the solidified portions SA are composed of a pure metal, segregation does not occur. Further, even when the solidified portions SA are composed of an alloy, segregation may not substantially occur depending on the composition and the cooling conditions in the additive manufacturing process. Note that, the cooling rate depends on conditions such as the energy density of the beam, the scanning speed, and the scanning pattern in the additive manufacturing process. Thus, in a case where segregation does not substantially occur, cells or cell interfaces, which are described later, are not formed in the solidified portions SA. However, such solidified portions SA can also be oriented to different crystallographic directions with respect to a predetermined direction. Therefore, mechanical properties that differ depending on the region in the inorganic structure 1 can be obtained.

Further, although in FIG. 2 and FIG. 3, as one example, the solidified portions SA1 and SA3 and the solidified portion SA2 have different preferential orientations in the build direction Z, the combination of crystallographic directions and in which direction they are oriented in the inorganic structure is not particularly limited. The plurality of solidified portions SA may have different preferential orientations in the X direction, or may have different preferential orientations in the Y direction. The plurality of solidified portions SA may also have different preferential orientations in another direction that is different from the X direction, the Y direction, and the Z direction. That is, it suffices that the inorganic structure 1 has a first solidified portion that has a first crystallographic direction preferentially oriented in a predetermined direction, and a second solidified portion that has a second crystallographic direction preferentially oriented in the aforementioned predetermined direction that is a different direction from the first crystallographic direction.

### [Regarding microstructure of solidified portion SA of inorganic structure 1]

Preferably, the solidified portion SA includes a plurality of sub-solidified areas that are each surrounded by a plurality of molten pool interfaces, and each sub-solidified area includes a plurality of cells and a plurality of cell interfaces that are interfaces between cells that are adjacent to each other. FIG. 5 is a cross-sectional view illustrating the microstructure of the solidified portion SA of the inorganic structure 1 illustrated in FIG. 2. The cross-sectional view in FIG. 5 is a cross-sectional view perpendicular to a beam scanning direction in a case where the inorganic structure 1 is manufactured by an additive manufacturing process to be described later.

Referring to FIG. 5, the solidified portion SA includes a plurality of sub-solidified areas SSA. A molten pool interface MI is formed between adjacent sub-solidified areas SSA. In other words, the sub-solidified area SSA is surrounded by a plurality of molten pool interfaces MI. Each sub-solidified area SSA includes a plurality of cells CE, and cell interfaces CI that are interfaces between adjacent cells. The term "cell CE" refers to one of the solidification morphologies when a molten inorganic material solidifies, and includes dendrites that do not have secondary arms. Note that, the solidification morphologies transition in the order of planar, cell, columnar dendrite, and equiaxed dendrite.

As described above, the inorganic structure 1 is integrally formed by an additive manufacturing process of additive manufacturing (AM). In the additive manufacturing process, the solidified portions SA are formed by melting and solidifying inorganic powder particles. The cooling rate during solidification in the additive manufacturing process is extremely fast. Therefore, in the solidified structure of the sub-solidified area SSA in the solidified portion SA, a plurality of cells CE extending in the growth direction, and cell interfaces CI which are formed between adjacent cells CE are formed.

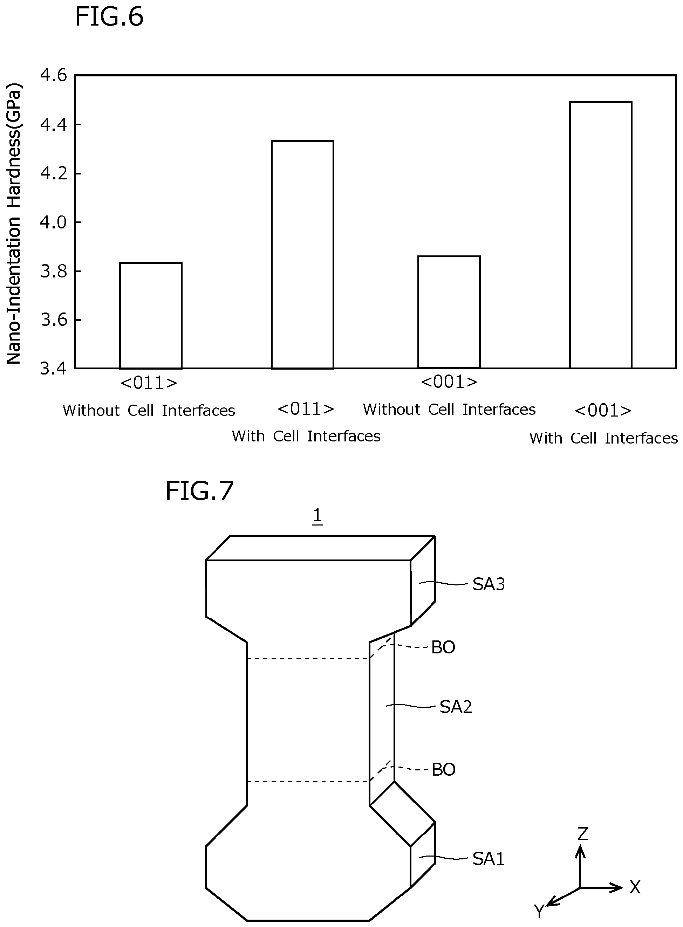

The solidified portion SA that includes the sub-solidified areas SSA including the plurality of cells CE and the cell interfaces CI exhibits extremely high plastic deformation resistance. FIG. 6 is a diagram illustrating the relation between the presence or absence of the cell interfaces CI in a solidified portion and the hardness (GPa) measured by nano-indentation. In FIG. 6, Inconel (registered trademark) 718 that is a Ni-based alloy with a cubic crystalline structure was used as the inorganic structure 1.

Referring to FIG. 6, in a case where the crystallographic direction in the build direction Z of the solidified portions SA is <011>, the hardness of a solidified portion SA in which cell interfaces CI are present is 13% higher than the hardness of a solidified portion SA in which there are no cell interfaces CI. Further, in a case where the crystallographic direction in the build direction Z of the solidified portion SA is <001> also, the hardness of a solidified portion SA in which cell interfaces CI are present is 16% higher than the hardness of a solidified portion SA in which there are no cell interfaces CI.

The sub-solidified area SSA of the inorganic structure 1 includes a plurality of the cells CE and the cell interfaces CI. Therefore, high mechanical properties can be obtained.

As described above, the inorganic structure 1 has a plurality of solidified portions SA, and the plurality of solidified portions SA include solidified portions SA that have different preferential orientations from each other. Therefore, the inorganic structure 1 can have mechanical properties that differ locally.

### [Second Embodiment]

In the inorganic structure 1 of the first embodiment, the plurality of solidified portions SA have the same shape. However, the plurality of solidified portions SA of the inorganic structure 1 may have different shapes from each other.

FIG. 7 is a perspective view of an inorganic structure 1 according to a second embodiment. Referring to FIG. 7, the inorganic structure 1 includes a plurality of solidified portions SA1 to SA3, and boundaries BO formed between adjacent solidified portions SA. In the inorganic structure 1, among the plurality of solidified portions SA, the solidified portion SA1 and the solidified portion SA2 have mutually different preferential orientations, similarly to the first embodiment.

In the inorganic structure 1 in FIG. 7, in addition, the solidified portions SA1 and SA2 have mutually different shapes. Therefore, in the inorganic structure 1 of the second embodiment, the solidified portions SA1 and SA2 have mutually different preferential orientations, and have mutually different shapes. For this reason, the mechanical properties obtained in the solidified portion SA1 are different from the mechanical properties obtained in the solidified portion SA2 due to the differences with respect to the shape and crystallographic orientation.

For example, let us assume that, in FIG. 7, <111> is preferentially oriented in the build direction Z of the solidified portions SA1 and SA3, and <001> is preferentially oriented in the build direction Z of the solidified portion SA2. In addition, in FIG. 7, a cross section perpendicular to the build direction Z of each of the solidified portion SA1 and the solidified portion SA3 is larger than a cross section perpendicular to the build direction Z of the solidified portion SA2. Therefore, the rigidity of the solidified portions SA1 and SA3 is markedly greater as compared to the rigidity of the solidified portion SA2.

### [Third embodiment]

In the first embodiment, the sub-solidified areas SSA of the inorganic structure 1 include a plurality of cells CE and a plurality of cell interfaces CI. However, the microstructure of the sub-solidified areas SSA of the inorganic structure 1 may be of a different form the first embodiment.

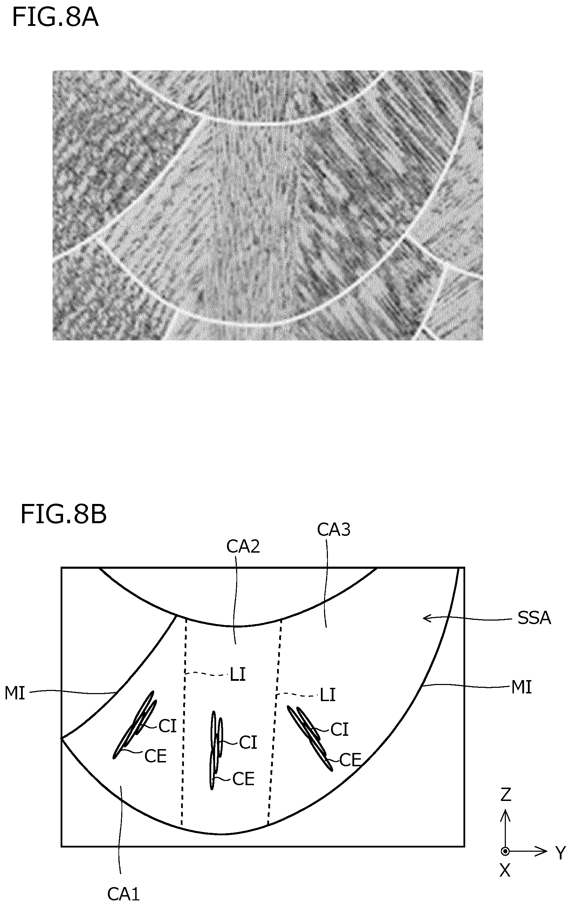

FIG. 8A is a photographic image showing the microstructure of a sub-solidified area SSA that is different from FIG. 5, and FIG. 8B is a schematic diagram of the photographic image in FIG. 8A. Referring to FIG. 8A and FIG. 8B, the sub-solidified area SSA includes a plurality of cell areas CA (CA1 to CA3). Each cell area CA includes a plurality of cells CE, and a plurality of cell interfaces CI formed between adjacent cells CE. The plurality of cells CE within the cell area CA grow along the same direction. That is, the plurality of cells CE within the cell area CA extend along the same direction.

With respect to the cell areas CA1 and CA2 that are adjacent, the extending direction of the cells CE in one cell area CA1 intersects with the extending direction of the cells CE in the other cell area CA2. A lamellar interface LI is formed between the adjacent cell areas CA1 and CA2. At the lamellar interface LI, the cell area CA1 that is one of the adjacent cell areas CA1 and CA2 has a preferential crystallographic orientation that is different from the crystallographic orientation of the other cell area CA2. The same also applies with respect to the cell areas CA2 and CA3 that are adjacent.

If the lamellar interface LI is formed in the sub-solidified area SSA, a crystallographic orientation difference at the atomic level is formed at the lamellar interface LI, and solute segregation occurs at the atomic level. Such kind of minute crystallographic orientation differences and minute amounts of solute displacement at the atomic level enhance mechanical properties such as the strength of the sub-solidified area SSA. As a result, the mechanical properties of the inorganic structure 1 increase.

### [Fourth embodiment]

In the first embodiment, the sub-solidified areas SSA of the inorganic structure 1 include a plurality of cells CE and a plurality of cell interfaces CI. However, the sub-solidified areas SSA of the inorganic structure 1 may be of a different form the first embodiment.

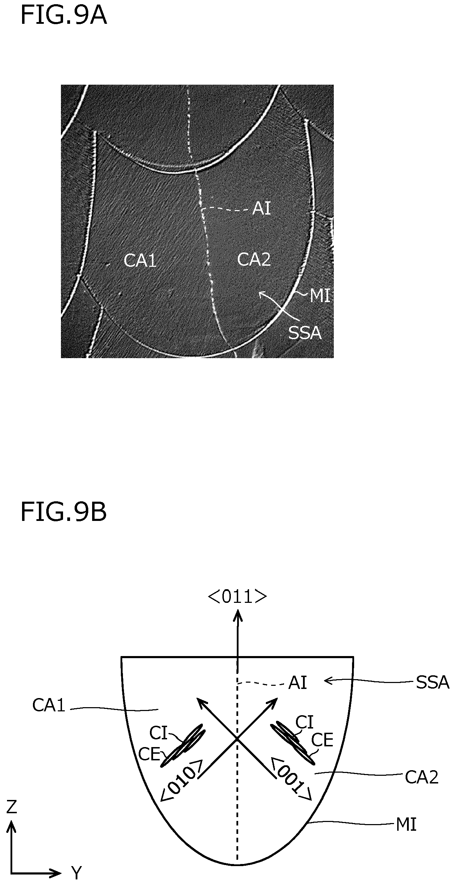

FIG. 9A is a photographic image showing the microstructure of a sub-solidified area SSA that is different from FIG. 5 and FIG. 8A and FIG. 8B. FIG. 9B is a schematic diagram of the photographic image in FIG. 9A. Referring to FIG. 9A and FIG. 9B, the sub-solidified area SSA includes a plurality of cell areas CA (CA1 and CA2). Each of the cell areas CA1 and CA2 includes a plurality of cells CE, and a plurality of cell interfaces CI formed between adjacent cells CE. The plurality of cells CE in the respective cell areas CA1 and CA2 grow (extend) along the same direction. In FIG. 9B, the plurality of cells CE in the cell area CA1 extend in the <010> direction. The plurality of cells CE in the cell area CA2 extend in the <001> direction.

An association interface AI is formed between the adjacent cell areas CA1 and CA2. As described above, in the adjacent cell areas CA1 and CA2, the extending direction <010> of the cells CE of the cell area CA1 intersects with the extending direction <001> of the cells CE of the other cell area CA2. Therefore, at the association interface AI, the cell area CA1 that is one of the adjacent cell areas CA1 and CA2 has the same crystallographic orientation in which <011> direction is preferentially oriented as the crystallographic direction of the other cell area CA2. Here, the term "same crystallographic orientation" means that an orientation difference between the crystallographic direction which the one cell area CA1 has and the crystallographic direction which the other cell area CA2 has is 10° or less.

As described above, at the association interface AI, the cell area CA1 that is one of the adjacent cell areas CA1 and CA2 has the same preferential crystallographic orientation as the other cell area CA2. If the association interface AI is formed in the sub-solidified area SSA, solute segregation at the atomic level occurs at the association interface AI. Such kind of minute amount of solute displacement at the atomic level enhances the mechanical properties such as the strength of the sub-solidified area SSA. As a result, the mechanical properties of the inorganic structure 1 increase.

For example, in the case of manufacturing the inorganic structure 1 using an additive manufacturing process to be described later, in the manufacturing process, as solidified layers which are described later are sequentially built, a difference between the crystal orientations at the association interface AI is self-adjusted so as to decrease. As a result, at the association interface AI of the manufactured inorganic structure 1, the crystallographic orientations of the adjacent cell areas CA1 and CA2 align. That is, the adjacent cell areas CA1 and CA2 become a single crystal or a single-crystal-like structure. The crystallographic orientation is inherited in the build direction Z by epitaxial growth. As a result, the orientation intensity of the crystallographic orientation in the build direction Z of the solidified portion SA can be further increased.

### [Regarding sub-solidified area SSA in a case where crystallographic direction orients in build direction Z is <011>]

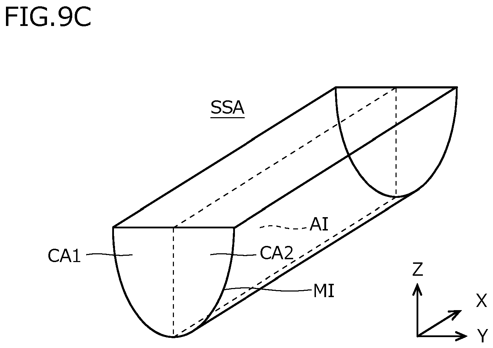

In FIG. 9A and FIG. 9B, the cells CE in the cell area CA1 extend in the <010> direction, and the cells CE in the cell area CA2 extend in the <001> direction. Therefore, the association interface AI where the cell area CA1 and the cell area CA2 come in contact extends in the build direction Z, and the extending direction of the association interface AI corresponds to <011>. Thus, in FIG. 9A and FIG. 9B, the sub-solidified area SSA constitutes a single crystal or a single-crystal-like structure, and the crystallographic direction in the build direction Z is <011>. Referring to FIG. 9A and FIG. 9B, the association interface AI extends in the build direction Z. In addition, as illustrated in FIG. 9C, the association interface AI extends in the X direction that is the extending direction of the sub-solidified area SSA. The sub-solidified area SSA extends along a scanning line on which a beam moves in an additive manufacturing process to be described later. Therefore, the crystallographic direction preferentially oriented in the build direction Z of the solidified portion SA which is composed of a plurality of sub-solidified areas SSA is <011>.

### [Regarding sub-solidified area SSA in a case where crystallographic direction oriented in build direction Z is <001>]

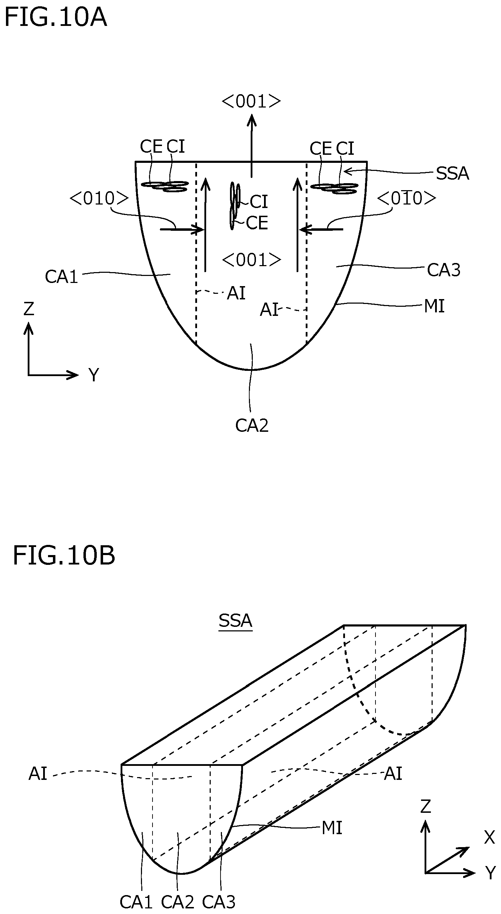

FIG. 10A and FIG. 10B are schematic diagrams of the microstructure of the sub-solidified area SSA in a case where the crystallographic direction oriented in the build direction Z is <001>. Referring to FIG. 10A, the sub-solidified area SSA includes three cell areas CA1, CA2, and CA3. In the cell area CA1, the cells CE extend in the <010> direction. In the cell area CA3, the cells CE extend in the <0-10> direction. The cell area CA2 is disposed between the cell area CA1 and the cell area CA3. In the cell area CA2, the cells CE extend in the <001> direction. Accordingly, in the cell areas CA1, CA2, and CA3, the crystallographic orientations match. Therefore, the sub-solidified area SSA constitutes a single crystal or a single-crystal-like structure. As a result, a plurality of association interfaces AI between the adjacent cell areas CA (CA1 to CA3) extend in the build direction Z, and extend in the <001> direction. In addition, as illustrated in FIG. 10B, two association interfaces AI extend in the X direction that is the extending direction of the sub-solidified area SSA. Therefore, the crystallographic direction preferentially oriented in the build direction Z of the solidified portion SA constituted by a plurality of the sub-solidified areas SSA is <001>.

### [Regarding sub-solidified area SSA in a case where crystallographic direction oriented in build direction Z is <111>]

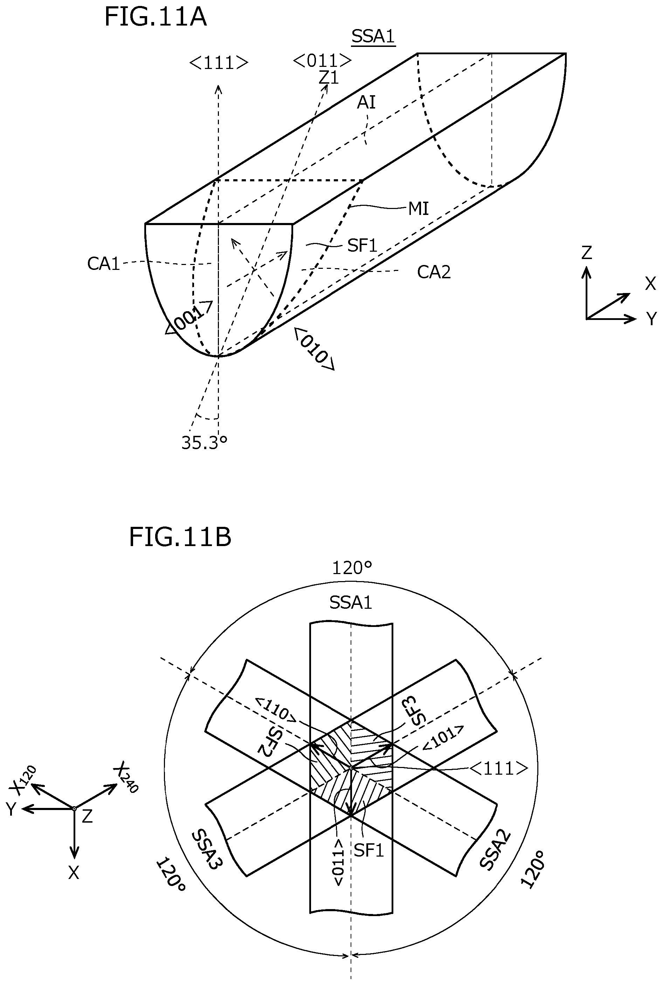

FIG. 11A is a perspective view of a sub-solidified area SSA1 in case where the crystallographic direction oriented in the build direction Z is <111>. Referring to FIG. 11A, the sub-solidified area SSA1 includes two cell areas CA1 and CA2. At a first surface SF1 which is inclined at 35.3° in the X direction from the build direction Z, the cells CE of the cell area CA1 extend in the direction of <001> which is inclined at 45° with respect to <011> which is inclined at 35.3° from the build direction Z. The cells CE of the cell area CA2 extend in the direction of <010> which is inclined at 45° with respect to <011>. Therefore, in the sub-solidified area SSA1, the crystallographic orientations of the cell areas CA1 and CA2 match, and the association interface AI extends in the X direction that is the extending direction of the sub-solidified area SSA1. In addition, at the first surface SF1, the association interface AI extends in the <011> direction.

FIG. 11B is a schematic diagram illustrating the inorganic structure 1 in which the crystallographic direction oriented in the build direction Z is <111>, as viewed from the build direction Z. Referring to FIG. 11B, a sub-solidified area SSA2 which is one layer above the sub-solidified area SSA1 extends in an X₁₂₀ direction that is a direction rotated by 120° from the X direction about the build direction Z with respect to the sub-solidified area SSA1. Similarly to the sub-solidified area SSA1, the sub-solidified area SSA2 includes two cell areas CA, which are not illustrated in the drawing. At a second surface SF2 which is inclined at 35.3° in the X₁₂₀ direction from the build direction Z and which is orthogonal to the first surface SF1, the cells CE of one of the cell areas CA extend in the <100> direction that is inclined at 45° with respect to the <110> direction that is inclined at 35.3° from the build direction Z. Further, the cells CE of the other cell area CA extend in the <010> direction that is inclined at 45° with respect to the <110> direction. Therefore, in the sub-solidified area SSA2, the crystallographic orientations of the two cell areas CA are matching, and the association interface AI extends in the X₁₂₀ direction. In addition, at the second surface SF2, the association interface AI extends in the <110> direction.

Further, a sub-solidified area SSA3 which is one layer above the sub-solidified area SSA2 extends in an X₂₄₀ direction that is a direction rotated by 240° from the X direction about the build direction Z with respect to the sub-solidified area SSA1. Similarly to the sub-solidified area SSA1, the sub-solidified area SSA3 includes two cell areas CA, which are not illustrated in the drawing. At a third surface SF3 which is inclined at 35.3° in the X₂₄₀ direction from the build direction Z and which is orthogonal to the first surface SF1 and the second surface SF2, the cells CE of one of the cell areas CA extend in the <100> direction that is inclined at 45° with respect to the <101> direction that is inclined at 35.3° from the build direction Z, and the cells CE of the other cell area CA extend in the <001> direction that is inclined at 45° with respect to the <101> direction. Therefore, in the sub-solidified area SSA3, the crystallographic orientations of the two cell areas CA are matching, and the association interface AI extends in the X₂₄₀ direction. In addition, at the third surface SF3, the association interface AI extends in the <101> direction.

As a result being composed as described above, each of the sub-solidified areas SSA1 to SSA3 constitutes a single crystal or a single-crystal-like structure, and the crystallographic direction oriented in the build direction Z is <111>. As a result, on the YZ plane in the sub-solidified area SSA, the association interface AI extends in the build direction Z, and extends in the <111> direction. Therefore, <111> preferentially orients in the build direction Z of the solidified portion SA constituted by the plurality of sub-solidified areas.

As described above, any one of the <001>, <011>, and <111> directions can be preferentially oriented in the build direction Z of each solidified portion SA constituting the inorganic structure 1. Accordingly, among the plurality of solidified portions SA of the inorganic structure 1, in one solidified portion SA, any one crystallographic direction among <001>, <011>, and <111> can be preferentially oriented in the build direction Z, and in another solidified portion SA, any one of <001>, <011>, and <111> and which is a different crystallographic direction to the crystallographic direction of the one solidified portion SA can be preferentially oriented in the build direction Z. Thus, the inorganic structure 1 can have different mechanical properties depending on the region in the inorganic structure 1.

Preferably, the inorganic structure 1 includes a first solidified portion and a second solidified portion, in which the crystallographic direction oriented in the build direction Z of the first solidified portion is <111>, and the crystallographic direction oriented in the build direction Z of the second solidified portion is <001> or <011> that is a different crystallographic direction from <111>. In this case, the mechanical properties can be made to differ between the first solidified portion and the second solidified portion.

### [Fifth embodiment]

In the first embodiment, the inorganic structure 1 includes a plurality of solidified portions SA. However, the inorganic structure 1 may include a structural unit of a form that is different from the plurality of solidified portions SA.

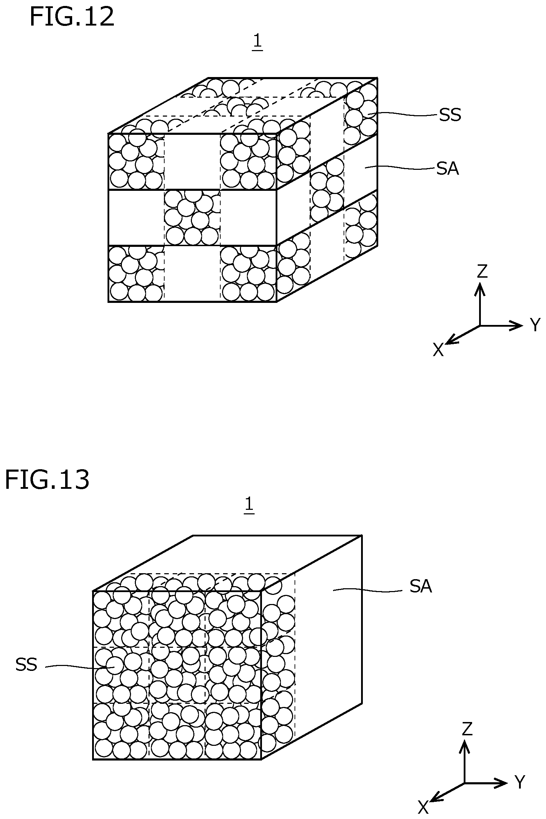

FIG. 12 is a perspective view of an inorganic structure 1 of an embodiment that is different from FIG. 2. As illustrated in FIG. 12, the inorganic structure 1 may include solidified portions SA and sintered portions SS. In this case, by adjusting the arrangement of the solidified portions SA and the sintered portions SS in the inorganic structure 1, the mechanical properties of the inorganic structure 1 can be adjusted so as to differ depending on the region, or the mechanical properties can be made anisotropic.

The sintered portion SS is formed by sintering inorganic powder particles by adjusting the energy density of a beam (a laser or an electron beam) in the additive manufacturing process. The sintered portion SS may also be formed by subjecting the inorganic structure 1 after additive manufacturing to a heat treatment. Specifically, during additive manufacturing, a beam is irradiated onto a region corresponding to the solidified portion SA, and irradiation of a beam is stopped in a region corresponding to the sintered portion SS. Thereafter, the inorganic structure 1 is subjected to a heat treatment to form the sintered portion SS.

The sintered portion SS is porous. Therefore, in the sintered portion SS, the strength is lower than in the solidified portion SA. For example, in the inorganic structure 1, the solidified portion SA is arranged at a region where high strength is required, and the sintered portion SS is arranged at a region where low strength is required. In this case, the inorganic structure 1 can have mechanical properties that differ depending on the region in the inorganic structure 1.

FIG. 13 is a perspective view of an inorganic structure 1 of a different embodiment from FIG. 12. Referring to FIG. 13, the inorganic structure 1 includes sintered portions SS and solidified portions SA. The solidified portions SA and the sintered portions SS are built in the X direction. In the solidified portion SA, the <111> crystallographic direction is preferentially oriented in the X direction.

Let us assume a case where the inorganic structure 1 having the above configuration is applied to the front part of the body of an automobile. At such time, the inorganic structure 1 is arranged in the automobile in a manner so that the sintered portions SS are arranged on the front side of the automobile, and the solidified portions SA are arranged on the cabin side. If the automobile receives an impact in the X direction from the front, the sintered portions SS of the inorganic structure 1 will plastically deform in response to the impact and thereby absorb the impact. On the other hand, in a region of the front part of the automobile that is near the cabin, the solidified portions SA in which <111> is preferentially oriented in the X direction are arranged. The solidified portions SA have high strength with respect to the <111> orientation. Therefore, the solidified portions SA are not easily deformed by an impact from the X direction, and the occurrence of a situation where the front part of the automobile deforms and penetrates into the cabin can be suppressed.

As described above, by the inorganic structure 1 including not only the solidified portions SA, but also the sintered portions SS, the inorganic structure 1 is given mechanical properties that differ depending on the region in the inorganic structure 1.

### [Sixth embodiment]

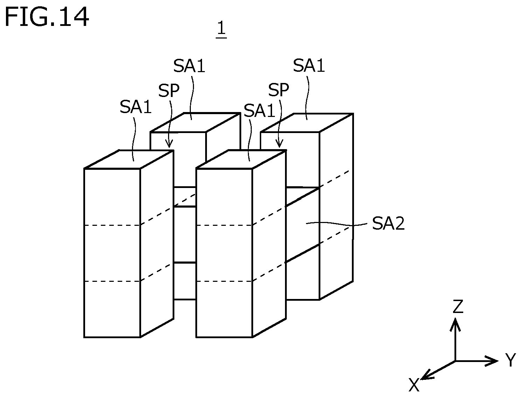

FIG. 14 is a perspective view of an inorganic structure 1 of a sixth embodiment. Referring to FIG. 14, the inorganic structure 1 has a space SP between adjacent solidified portions SA. Specifically, the inorganic structure 1 includes a plurality of solidified portions SA1 extending in the build direction Z, and a solidified portion SA2 which extends in the Y direction and which is in contact with a central part in the build direction Z of each of the plurality of solidified portions SA1. The solidified portions SA1 and the solidified portion SA2 are integrally formed. In the inorganic structure 1 in FIG. 14, a space SP is formed between adjacent solidified portions SA1. In the solidified portion SA1, <111> is preferentially oriented in the build direction Z. On the other hand, in the solidified portion SA2, <001> is preferentially oriented in the build direction Z.

The inorganic structure 1 including the spaces SP is not limited to the shape illustrated in FIG. 14. The shape of the inorganic structure 1 is not particularly limited as long as the inorganic structure 1 includes a plurality of solidified portions SA, and a plurality of spaces SP which are formed between adjacent solidified portions SA. As illustrated in FIG. 14, the space SP may be a space in which one part is open, or may be a closed space that is surrounded by solidified portions SA.

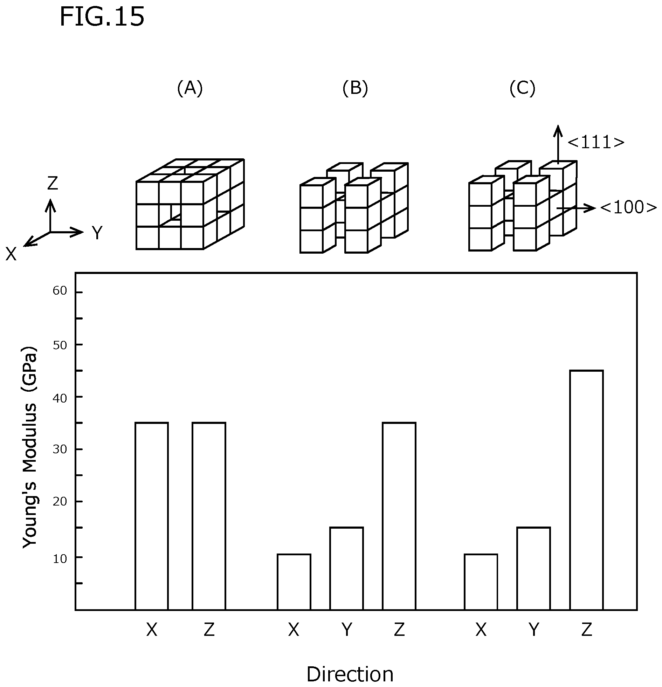

In a case where the space SP is formed between adjacent solidified portions SA, differences in the mechanical properties that depend on the region in the inorganic structure 1 can be further increased. FIG. 15 is a diagram illustrating the relation between various inorganic structures 1 having different shapes and crystallographic orientations, and Young's modulus in the X direction, Y direction, and Z direction. Referring to FIG. 15, an inorganic structure (A) is a cube in which a through-hole is formed at the center position of each surface. In addition, in the inorganic structure (A), the solidified portions SA are composed of polycrystals. An inorganic structure (B) has the same shape as the inorganic structure 1 shown in FIG. 14. However, the solidified portions SA are composed of polycrystals. An inorganic structure (C) has the same shape and the same crystallographic orientation as the inorganic structure 1 shown in FIG. 14.

Referring to FIG. 15, in the inorganic structure (A) whose shape is isotropic in the X direction and the Z direction, the Young's modulus in the X direction and the Young's modulus in the Z direction are approximately the same. On the other hand, the inorganic structure (B) has different shapes as viewed from the X direction, Y direction, and Z direction, respectively, and is anisotropic in shape. Consequently, the Young's modulus in the X direction, the Young's modulus in the Y direction, and the Young's modulus in the Z direction of the inorganic structure (B) are significantly different from each other. Specifically, the Young's modulus in the X direction is lowest, and the Young's modulus in the Z direction is highest. Further, with respect to the inorganic structure (C), the inorganic structure (C) not only has an anisotropic shape, but also has anisotropic crystallographic orientations. Therefore, in the inorganic structure (C), the Young's modulus in the Z direction is significantly increased compared to the inorganic structure (B).

As described above, by having shape anisotropy and crystallographic orientation anisotropy, the inorganic structure 1 of the present embodiment can have mechanical properties that differ significantly from region to region in the inorganic structure 1.

### [Other embodiments]

The solidified portion SA of the inorganic structure 1 described in the above embodiments may be a polycrystal, or may be a single-crystal-like structure or a single crystal. Preferably, the solidified portion SA is a single-crystal-like structure or a single crystal. In this case, the orientation intensity of a specific crystallographic orientation can be increased. Therefore, it will be easier to adjust the mechanical properties that differ depending on the region. Here, the term "single-crystal-like structure" means, for example, in a case where the crystalline structure of the inorganic structure 1 is a cubic crystalline structure, a structure in which {001} poles are concentrated into three in a {001} pole figure.

As described above, the inorganic structure 1 of the present embodiment includes a first solidified portion that has a crystallographic orientation in which a first crystallographic direction is preferentially oriented in a predetermined direction, and a second solidified portion that has a crystallographic orientation in which a second crystallographic direction that is a different direction from the first crystallographic direction is preferentially oriented in the predetermined direction. In the inorganic structure 1, by including the plurality of solidified portions in which the crystallographic orientation are different, mechanical properties that differ depending on the region in the inorganic structure 1 are obtained. The composition of this kind of inorganic structure 1 is not particularly limited as long as the structure is composed of an inorganic material. For example, the inorganic structure 1 may have a composition consisting of a pure metal, may have a composition consisting of an alloy, or may have a composition consisting of a non-metallic inorganic material such as a ceramic material. Note that the predetermined direction is not particularly limited. The predetermined direction may be the build direction Z, or may be another direction.

### [Method for manufacturing inorganic structure 1]

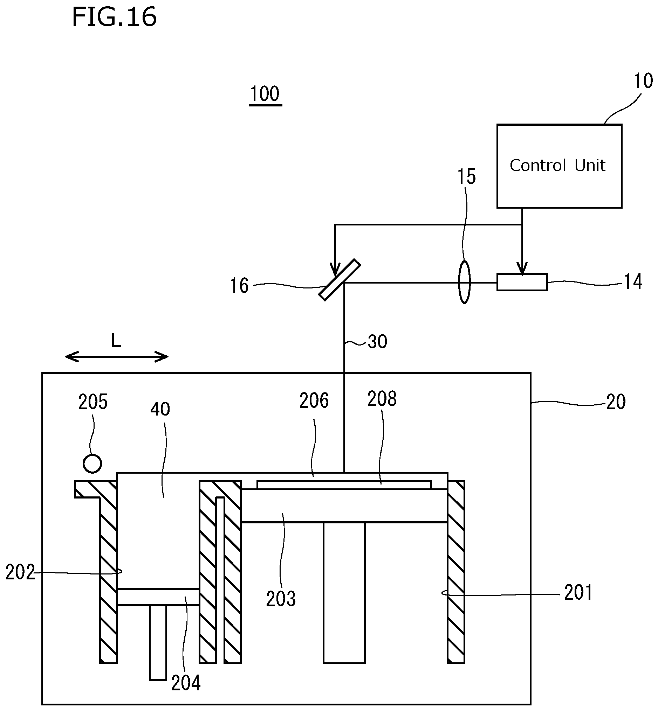

Hereunder, a method for manufacturing the inorganic structure 1 is described. In the method for manufacturing the inorganic structure 1, for example, an additive manufacturing device illustrated in FIG. 16 is used.

### [Configuration of additive manufacturing device]

Referring to FIG. 16, an additive manufacturing device 100 is a laser additive manufacturing device. The additive manufacturing device 100 includes a control unit 10, a beam generator 14, a lens system 15, a mirror (galvanometer mirror) 16, and a chamber 20.

The beam generator 14 is a high energy heat source, and emits a beam. Here, the term "beam" refers to a laser beam or an electron beam. The beam generator 14 is, for example, a solid-state laser such as a fiber laser or a YAG laser, or a CO₂ laser. The lens system 15 converges the beam emitted from the beam generator 14 to form a beam 30. The mirror 16 is used when performing an operation to irradiate the beam 30. That is, the position at which the beam 30 is irradiated is adjusted using the mirror 16.

The chamber 20 includes a bed forming chamber 201, a powder supply chamber 202, a modeling table 203, and a recoater 205.

The bed forming chamber 201 is cylindrical and has an opening at the upper end. The modeling table 203 is housed in the bed forming chamber 201, and is supported so as to be capable of moving up and down in the vertical direction. The modeling table 203 is moved up and down by a motor (not illustrated in the drawings). A base 208 is arranged on the modeling table 203. The base 208 is made of a metal such as, for example, titanium. The base 208 may also be made of a metal other than titanium. The inorganic structure 1 is formed on the base 208.

The powder supply chamber 202 is arranged next to the bed forming chamber 201. The powder supply chamber 202 has a cylindrical shape and includes therein a piston 204 that can move up and down in the vertical direction. Inorganic powder particles 40 that serve as the starting material of the inorganic structure 1 are stacked on the piston 204. When the piston 204 rises, the inorganic powder particles 40 are discharged from an upper opening of the powder supply chamber 202 and supplied to the bed forming chamber 201.

The recoater 205 is arranged in the vicinity of the upper opening of the powder supply chamber 202. The recoater 205 is moved in a specific direction (horizontal direction) by an unshown motor to move reciprocally between the powder supply chamber 202 and the bed forming chamber 201. In FIG. 16, the recoater 205 moves reciprocally in an L direction.

By moving in the L direction, the recoater 205 causes the inorganic powder particles 40 discharged from the powder supply chamber 202 to move in the horizontal direction to thereby supply the inorganic powder particles 40 to the bed forming chamber 201. An inorganic powder layer 206 is formed by the inorganic powder particles 40 accumulated on the base 208 of the modeling table 203 inside the bed forming chamber 201. As the recoater 205 moves in the L direction, the inorganic powder particles 40 move in the horizontal direction so that the surface of the inorganic powder layer 206 is made flat.

Note that, a roller may be used instead of the recoater 205. In a case where a roller is used, the inorganic powder layer 206 can be formed while pressing the roller against the inorganic powder particles 40 as desired.

The control unit 10 includes a central processing unit (CPU), a memory, and a storage that is typified by an HDD or an SSD that are not illustrated in the drawings. A well-known CAD (Computer Aided Design) application and a CAM (Computer Aided Manufacturing) application are stored in the storage.

The control unit 10 utilizes the well-known CAD application to set a three-dimensional shape of the inorganic structure 1 to be manufactured. In addition, the control unit 10 utilizes the CAM application to set processing conditions based on the three-dimensional shape that was set. In the additive manufacturing process, a plurality of solidified layers formed by the beam 30 are built to manufacture the inorganic structure 1. The processing conditions include processing conditions to be used when forming the respective solidified layers. The control unit 10 controls the beam generator 14, the lens system 15, and the mirror 16 based on the processing conditions data to adjust the energy density, scanning speed, scanning pattern, and beam irradiation position of the beam 30.

### [Regarding manufacturing process]

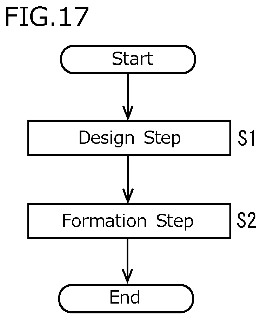

The method for manufacturing the inorganic structure 1 of the present embodiment manufactures the inorganic structure 1 using an additive manufacturing process. FIG. 17 is a flowchart of the method for manufacturing the inorganic structure 1 of the present embodiment. Referring to FIG. 17, the manufacturing method of the present embodiment includes a design step S1 and a formation step S2. In the design step S1, a three-dimensional shape and orientations of the crystallographic orientations of the inorganic structure 1 are determined. Further, the beam scanning method is set based on the orientations of the crystallographic orientations. In the formation step, the beam is scanned based on the beam scanning method that was set, and the inorganic powder particles 40 are thereby melted to manufacture the inorganic structure 1 including a plurality of solidified portions SA preferentially oriented to different crystallographic orientations. Hereunder, the design step S1 and the formation step S2 are described in detail.

### [Regarding design step S1]

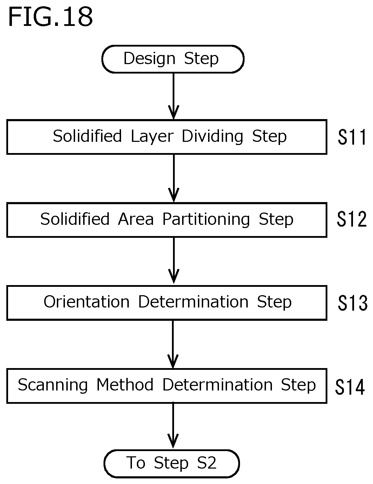

FIG. 18 is a flowchart of the design step S1. Referring to FIG. 18, first, the desired three-dimensional shape of the inorganic structure 1 is determined using the control unit 10. In the present example, the description will be continued assuming that the inorganic structure 1 is to have a cubic shape.

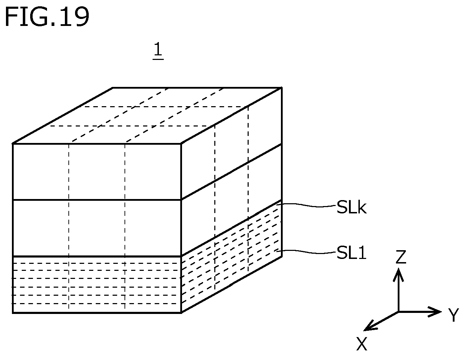

After the three-dimensional shape of the inorganic structure 1 is determined, as illustrated in FIG. 19, the three-dimensional shape of the inorganic structure 1 is divided into a plurality of solidified layers SLk built in the build direction Z (S11). Specifically, the inorganic structure 1 is sliced in a direction j perpendicular to the build direction Z by a preset number of built layers kmax (layers) of solidified layers SLk (k is a natural number, and the maximum number is kmax) to thereby divide the inorganic structure 1 into a plurality of solidified layers SLk (k = 1 to kmax). In some cases, each of the plurality of solidified layers SLk of the inorganic structure 1 may have a different shape from the other solidified layers SLk. Therefore, the processing conditions data is set for each solidified layer SLk.

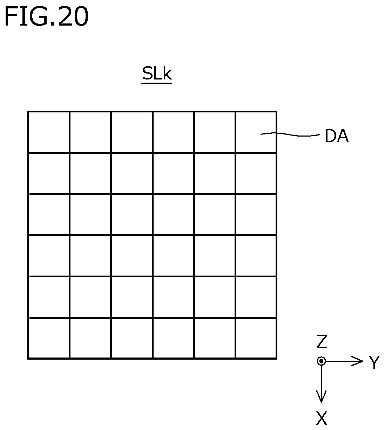

In addition, referring to FIG. 20, each solidified layer SLk is partitioned into a plurality of solidified areas DA as viewed in the build direction Z of the solidified layers SLk (S12). The solidified area DA is a unit area that serves as a standard size to which the scanning path with respect to the beam scanning is applied. In FIG. 20, the solidified area DA is a rectangular shape. However, the shape of the solidified area DA may be a different shape other than rectangular. For example, the solidified area DA may be a triangular shape or a polygonal shape other than a rectangle.

Next, the control unit 10 determines the orientation of the crystallographic orientation in each solidified area DA based on the mechanical properties required in each solidified area DA of each solidified layer SLk (S13). The control unit 10 then determines the scanning method (scanning path) for scanning the beam according to the determined orientations of the crystallographic orientations (S14).

For example, any one of <001>, <011>, and <111> is determined as the crystallographic orientation in the build direction Z in each solidified area DA (S13).

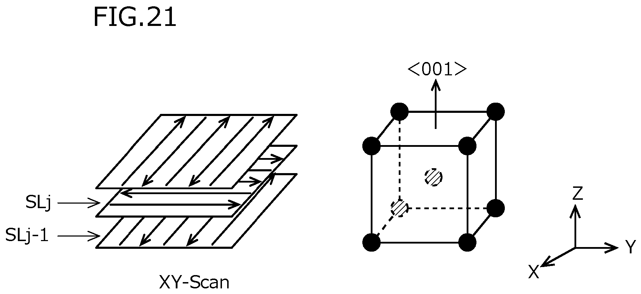

In a case where the determined crystallographic orientation is <001>, as illustrated in FIG. 21, in the solidified area DA for which the <001> crystallographic orientation was determined, the scanning direction of the beam when forming a solidified layer SLj that is a j-th layer (j is a natural number) is adjusted so as to be orthogonal to the scanning direction of the beam when forming a solidified layer SLj-1 that is a j-1th layer that is the layer underneath the j-th layer. In addition, in the solidified area DA, the beam is scanned in a reciprocating manner. As a result, the crystalline structure on an orthogonal coordinate system becomes the structure as illustrated in FIG. 21, in which the <001> direction is preferentially oriented in the build direction Z.

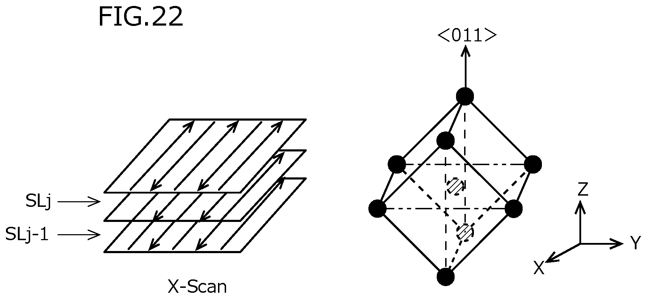

In a case where the selected crystallographic orientation is <011>, as illustrated in FIG. 22, in the solidified area DA for which the <011> crystallographic orientation was determined, the scanning direction of the beam when forming a solidified layer SLj that is the j-th layer is adjusted so as to be parallel to the scanning direction of the beam when forming a solidified layer SLj-1 that is the j-1th layer. In addition, in the solidified area DA, the beam is scanned in a reciprocating manner. As a result, the crystalline structure on an orthogonal coordinate system becomes the structure as illustrated in FIG. 22, in which the <011> direction is preferentially oriented in the build direction Z.

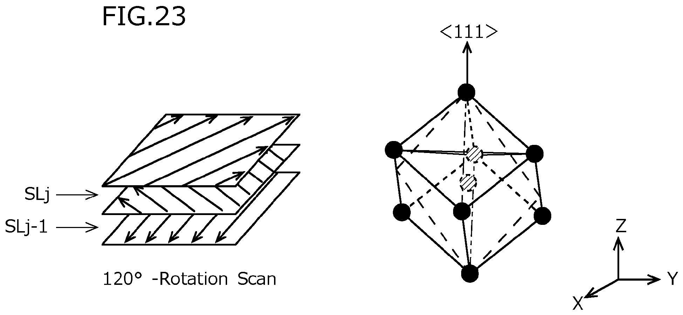

In a case where the selected crystallographic orientation is <111>, as illustrated in FIG. 23, in the solidified area DA for which the <111> crystallographic orientation was determined, the scanning direction of the beam when forming a solidified layer SLj that is the j-th layer is adjusted so as to intersect at 120° with respect to the scanning direction of the beam when forming a solidified layer SLj-1 that is the j-1th layer. In addition, the scanning direction of the beam when forming a solidified layer SLj+1 that is the j+1th layer is adjusted so as to intersect at 120° with respect to the scanning direction of the beam when forming the solidified layer SLj that is the j-th layer and to intersect at 240° with respect to the scanning direction of the beam when forming the solidified layer SLj-1 that is the j-1th layer, and furthermore, in each solidified area DA, the beam is not scanned in a reciprocating manner, and is scanned in only one direction. As a result, the crystalline structure on an orthogonal coordinate system becomes the structure as illustrated in FIG. 23, in which the <111> direction is preferentially oriented in the build direction Z. In the case of forming the solidified layers in sequence from the solidified layer SLj-1 that is j-1th layer to the solidified layer SLj+1 that is the j+1th layer, the beam scanning direction in each solidified layer may be rotated in increments of 120° in the clockwise direction in the order in which the solidified layers are built in plan view, or may be rotated in increments of 120° in the counter-clockwise direction in the order in which the solidified layers are built of the solidified layers in plan view.

By the above method, the crystallographic orientation in the build direction Z in each solidified area DA is determined (S13), and the method for scanning the beam is determined based on the determined crystallographic orientation (S14). Note that, in the step of determining the beam scanning method, beam conditions (output E of the beam 30, the scanning speed, and the like) are also determined according to the energy density (J/mm²) required for melting and solidifying the solidified area DA.

Information relating to beam scanning paths based on crystallographic orientations and the beam conditions is, for example, stored in advance in the storage in the control unit 10 in correspondence with the composition of the inorganic powder particles 40. By performing the above steps, processing conditions data for each solidified area DA of each solidified layer SLk is created. The plurality of pieces of processing conditions data that are created are stored, for example, in a memory inside the control unit 10.

### [Regarding formation step S2]

In the formation step S2, beam scanning is performed based on the processing conditions data determined in the design step S1 to thereby form each solidified layer SLk and manufacture the inorganic structure 1. Note that, the formation step S2 is performed after evacuating the interior of the chamber 20 of the additive manufacturing device 100.

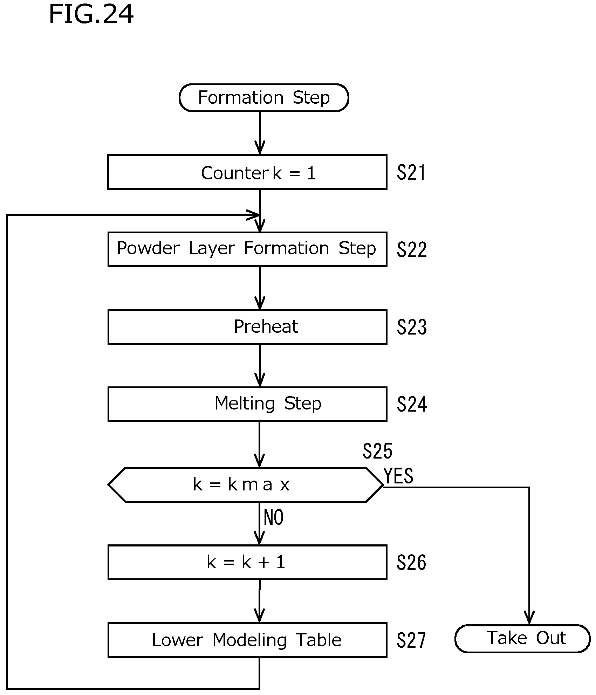

FIG. 24 is a flowchart of the formation step S2. Referring to FIG. 24, first, the control unit 10 sets a counter k to "1" (S21), and starts formation of a first solidified layer SL1 that is the bottom layer.

The control unit 10 supplies an inert gas (argon, nitrogen, helium, or the like) into the chamber 20, and preheats the base 208 arranged on the modeling table 203. Note that, during preheating, after creating a vacuum, the base 208 may be preheated without supplying inert gas into the chamber 20. Alternatively, the base 208 may be preheated after supplying inert gas into the chamber 20 without creating a vacuum. Alternatively, preheating of the base 208 need not be performed.

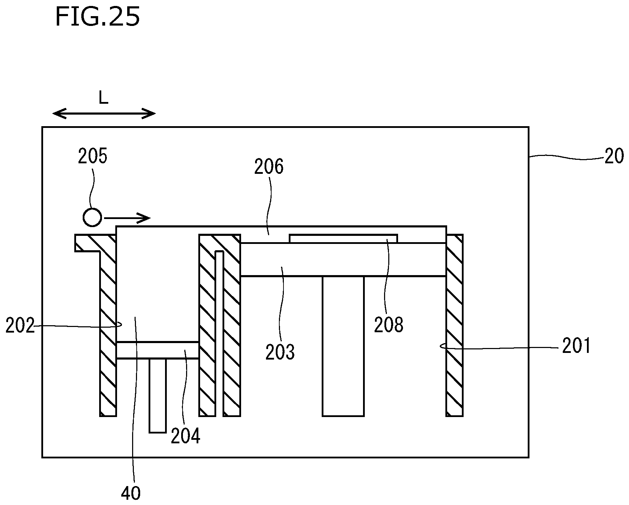

Next, the control unit 10 forms the inorganic powder layer 206 (S22). The control unit 10 issues a command to raise the piston 204 of the powder supply chamber 202 to discharge the inorganic powder particles 40 that will serve as the starting material of the inorganic structure 1. In response to the command from the control unit 10, the powder supply chamber 202 discharges the inorganic powder particles 40. At this time, as illustrated in FIG. 25, the recoater 205 moves in the L direction. By this means, the discharged inorganic powder particles 40 are moved in the L direction and supplied to the bed forming chamber 201. The inorganic powder particles 40 accumulate on the base 208 and the modeling table 203, and the inorganic powder layer 206 is formed.

The recoater 205 also moves horizontally (in the L direction) on the surface of the inorganic powder layer 206 of the base 208. At such time, the inorganic powder particles 40 move in the horizontal direction. The thickness of the inorganic powder layer 206 that is the bottom layer is, for example, around 5 to 1000 µm.

Next, the control unit 10 preheats the inorganic powder layer 206 (S23). Various methods can be used to preheat the inorganic powder layer 206. For example, the inorganic powder layer 206 may be preheated by irradiating the beam 30 thereon, or the inorganic powder layer 206 may be preheated by increasing the temperature of the modeling table 203. The inorganic powder layer 206 may also be preheated by heating the inorganic powder particles 40 with a heater inside the bed forming chamber 201.

Next, based on the beam scanning method determined in the design step S1, the inorganic powder layer 206 is scanned with the beam 30 to melt the inorganic powder layer 206 and form a solidified layer SL1 that is the first layer (S24). Among the plurality of pieces of processing conditions data created in the design step S1, the control unit 10 reads out the processing conditions data for the solidified layer SL1 that is the first layer from the memory. The control unit 10 controls the beam 30 based on the processing conditions data that is read out.

Specifically, the control unit 10 scans each solidified area DA with the beam 30 using a beam scanning path (beam scanning method) in accordance with the crystallographic orientation determined for each solidified area DA in the solidified layer SL1. By this means, the solidified layer SL1 is formed that is constituted by a plurality of solidified areas DA having the crystallographic orientations as determined in the design step S1.

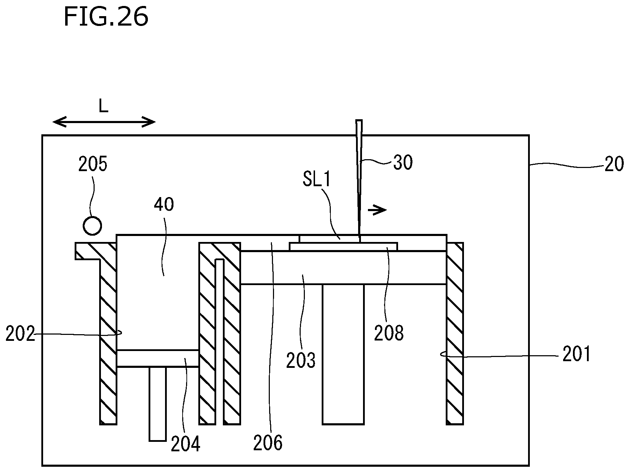

In the melting step (S24), not only the inorganic powder layer 206, but also a surface layer portion of the base 208 may be melted. The beam 30 is scanned under the aforementioned beam conditions to melt the inorganic powder layer 206 and form the solidified layer SL1 as illustrated in FIG. 26. At such time, in the inorganic powder layer 206, those inorganic powder particles 40 which are disposed in an area other than the solidified layer SL1 are not melted and are also not sintered.

After the solidified layer SL1 that is the first layer is formed, the control unit 10 determines whether or not the counter k is kmax (S25). Here, since the counter k = 1 (NO in S25), the control unit 10 sets the counter k to k+1 = 2 (S26). The control unit 10 then prepares to manufacture a solidified layer SL2 that will be the second layer.

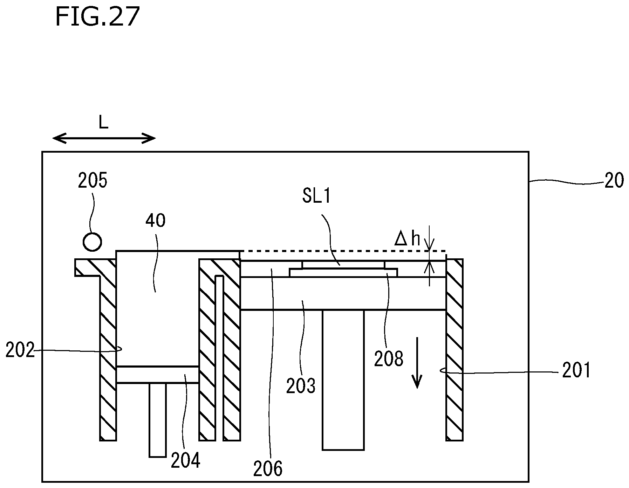

The control unit 10 lowers the modeling table 203 by the amount of a later thickness Δh (S27). As a result, as illustrated in FIG. 27, the surface of the inorganic powder layer 206 is lowered by an amount corresponding to Δh as compared to FIG. 26.

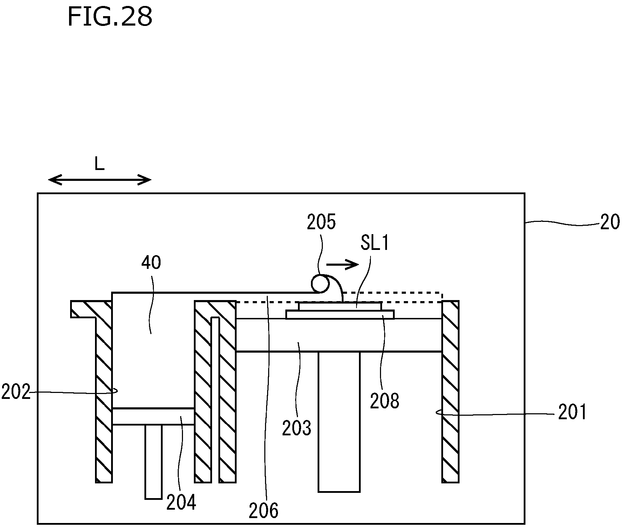

After step S27 is completed, the process returns to step S22. At such time, the control unit 10 forms a new inorganic powder layer 206 on the inorganic powder layer 206 where the solidified layer SL1 has been formed (S22). Specifically, in response to a command from the control unit 10, the piston 204 inside the powder supply chamber 202 rises and the inorganic powder particles 40 are discharged again. At such time, as illustrated in FIG. 28, the recoater 205 moves in the L direction. Therefore, the inorganic powder particles 40 move in the L direction to thereby form a new inorganic powder layer 206 having a thickness of Δh. The surface of the new inorganic powder layer 206 is made flat by the recoater 205.

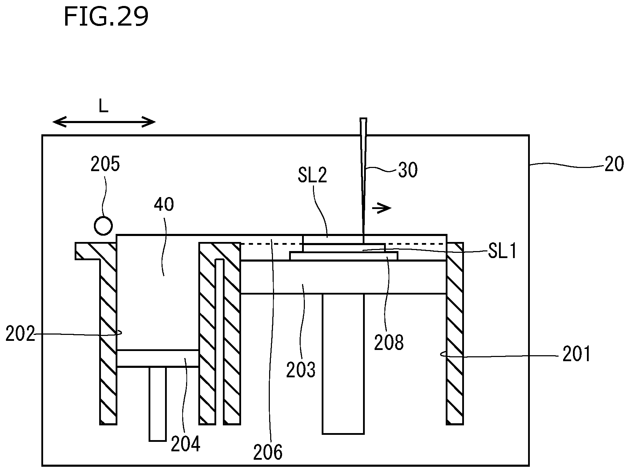

Next, the control unit 10 preheats the inorganic powder layer 206 (S23), and forms the solidified layer SL2 that is the second layer (S24). At such time, the control unit 10 irradiates the beam 30 onto the inorganic powder layer 206 based on the processing conditions data for the k-th layer (here, k = 2). As a result, as illustrated in FIG. 29, the inorganic powder layer 206 that is within the area irradiated with the beam 30 is melted and solidified, and the solidified layer SL2 is formed. By performing the above process, the solidified layer SL2 is built on the solidified layer SL1.

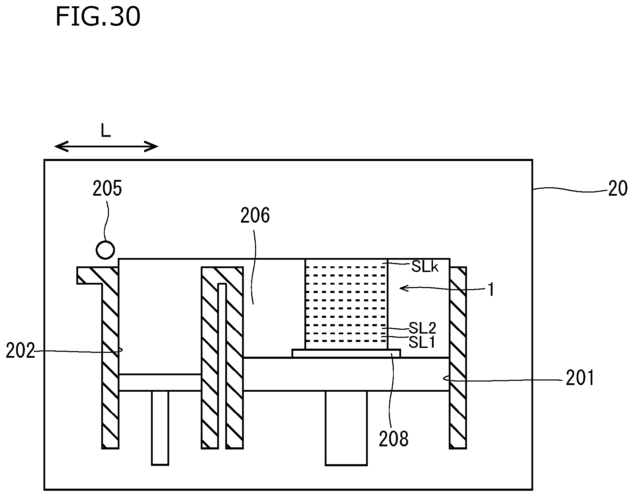

Next, the process proceeds to step S25, and until the value of k becomes kmax, in other words, until a solidified layer SLkmax that is the uppermost layer is formed, the control unit 10 repeats the operation from step S22 to step S27. That is, the control unit 10 repeats the layer formation step (S22) and the melting step (S24) until the inorganic structure 1 having the desired three-dimensional shape is completed as illustrated in FIG. 30.

Note that, the shape of the solidified layer SLk is not restricted to the shape of the underlying solidified layer SLk-1, and can be made a larger shape than the solidified layer SLk-1 or a smaller shape than the solidified layer SLk-1. In addition, one or a plurality of spaces can be formed in the shape of the solidified layer SLk. In this case, the structure can have a shape that cannot be obtained by normal cutting, such as a shape that has a closed space or a shape that has a complicated hole shape. The inorganic structure 1 of the present embodiment can be manufactured by the above manufacturing method.

### [Regarding a case where target solidified area DA is smaller than solidified area DA which serves as a standard]

As described above, in the manufacturing method of the present embodiment, a solidified area DA illustrated in FIG. 20 is used as a standard for determining a scanning method that includes a scanning path of the beam. That is, the scanning path of the beam is determined based on the solidified area DA having a size which serves as a standard.

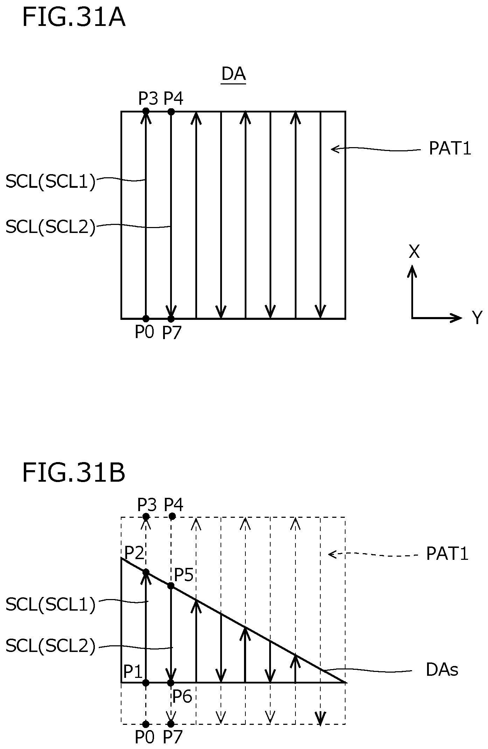

FIG. 31A is a diagram illustrating a scanning path of a beam within a solidified area of a size that serves as a standard, and FIG. 31B is a schematic diagram for describing a scanning path of a beam in a solidified area that is smaller in size than the solidified area having the size that serves as a standard.

As illustrated in FIG. 31A, it is assumed that in the solidified area DA of the standard size, the scanning path of the beam in a case where the preferential crystallographic direction in the build direction Z is <001> is PAT1. Here, the term "scanning path PAT1" means a path along which the beam irradiation position moves at a predetermined scanning speed in the solidified area DA serving as a standard. The scanning path PAT1 includes a plurality of scanning lines SCL. The scanning lines SCL are parallel to each other. The plurality of scanning lines SCL are arranged in a direction perpendicular to the direction in which the scanning lines SCL extend. While the beam irradiation position is moving at a predetermined scanning speed on the scanning path, the additive manufacturing device 100 irradiates the beam 30 onto the beam irradiation position. By this means, the beam 30 is irradiated onto the inorganic powder layer 206 along the scanning path to thereby melt the inorganic powder layer 206.

Specifically, in the case of applying the scanning path PAT1 to the solidified area DA of the standard size, the beam irradiation position is moved on a scanning line SCL1 at a predetermined scanning speed. While the beam irradiation position is moving on the scanning line SCL1, the additive manufacturing device 100 irradiates the beam 30 onto the beam irradiation position. After the beam irradiation position has moved the entire length of the scanning line SCL1, the additive manufacturing device 100 shifts the beam irradiation position to an end point of a scanning line SCL2 that is adjacent to the scanning line SCL1. Thereafter, the beam irradiation position is moved on the scanning line SCL2 at a predetermined scanning speed. While the beam irradiation position is moving on the scanning line SCL2, the additive manufacturing device 100 irradiates the beam 30 onto the beam irradiation position. The beam 30 is also irradiated in a similar manner on the scanning lines SCL other than the scanning lines SCL1 and SCL2 of the scanning path PAT1 to thereby irradiate the beam 30 over the entire length of the scanning path PAT1 and melt the solidified area DA.

On the other hand, when the solidified layer SLk is partitioned into a plurality of solidified areas DA of a standard size as illustrated in FIG. 20, in some cases, a solidified area DAs which has a smaller size than the solidified area DA of the standard size may be partitioned at an edge of the solidified layer SLk, such as in a case illustrated in FIG. 31B. When performing the melting step with respect to this kind of solidified area DAs that has a small size, the heat amount of the beam is adjusted by the following method.

Specifically, the beam is irradiated at a portion (solid line portion of PAT1) where the scanning path PAT1 overlaps with the solidified area DAs to be melted, and irradiation of the beam is stopped at a portion (dashed line portion of PAT1) where the scanning path PAT1 does not overlap with the solidified area DAs. That is, within the scanning path PAT1, during a period in which the beam irradiation position is moving at a predetermined scanning speed over a portion that overlaps with the solidified area DAs, the beam is irradiated onto the beam irradiation position, and a period in which the beam irradiation position is moving at a predetermined scanning speed over a portion that does not overlap with the solidified area DAs is set as an off time period in which irradiation of the beam is stopped.

Referring to FIG. 31B, in a case where the scanning path PAT1 for the solidified area DA of the standard size is applied to the solidified area DAs that is smaller than the solidified area DA that is the standard size, the scanning line SCL 1 does not overlap with the solidified area DAs within a range from an end point P0 to a point P1 on the scanning line SCL1. Therefore, during the period in which the beam irradiation position moves at the predetermined scanning speed from the end point P0 to the point P1, the additive manufacturing device 100 stops irradiation of the beam 30. That is, the time period in which the beam irradiation position moves from the end point P0 to the point P1 on the scanning line SCL1 is set as an off time period.

After the beam irradiation position reaches the point P1, within a range in which the beam irradiation position moves from the point P1 until arriving at a point P2, the scanning line SCL1 overlaps with the solidified area DAs. Therefore, during the period in which the beam irradiation position moves at the predetermined scanning speed from the point P1 to the point P2, the additive manufacturing device 100 irradiates the beam 30 onto the beam irradiation position.

After the beam irradiation position reaches the point P2, the scanning line SCL1 does not overlap with the solidified area DAs within a range in which the beam irradiation position moves from the point P2 until arriving at an end point P3. Therefore, the additive manufacturing device 100 sets the time period in which the beam irradiation position moves from the point P2 to the end point P3 as an off time period.

Similarly, for the scanning line SCL2, the additive manufacturing device 100 sets a time period in which the beam irradiation position moves from an end point P4 to a point P5, and a time period in which the beam irradiation position moves from a point P6 to an end point P7 as an off time period, respectively. Further, during the period in which the beam irradiation position moves at the predetermined scanning speed from the point P5 to the point P6, the additive manufacturing device 100 irradiates the beam 30 onto the beam irradiation position.

By means of the above method, for the scanning lines SCL other than the scanning lines SCL1 and SCL2 of the scanning path PAT1 also, the beam 30 is irradiated onto the beam irradiation position during a time period in which the beam irradiation position moves within a range that overlaps with the solidified area DAs, and a time period during which the beam irradiation position moves a range that does not overlap with the solidified area DAs is set as an off time period.

Thus, in a case where the solidified area DAs is smaller than the solidified area DA of the standard size, within the scanning path PAT1, the beam 30 is irradiated only onto the path at a portion where the solidified area DAs overlaps, and irradiation of the beam 30 is stopped (off time period) at portions of the path where the solidified area DAs does not overlap. In a case where an off time period is provided in this way, the thermal history applied to the solidified area DAs by the beam 30 becomes approximately the same as the thermal history applied to the standard-sized solidified area DA by the beam 30. Therefore, in the solidified area DAs whose size is smaller than the standard-sized solidified area DA also, a microstructure that is approximately the same as the microstructure of the solidified area DA can be formed, and the solidified area DAs can have a crystallographic orientation that is equal to the crystallographic orientation of the solidified area DA.

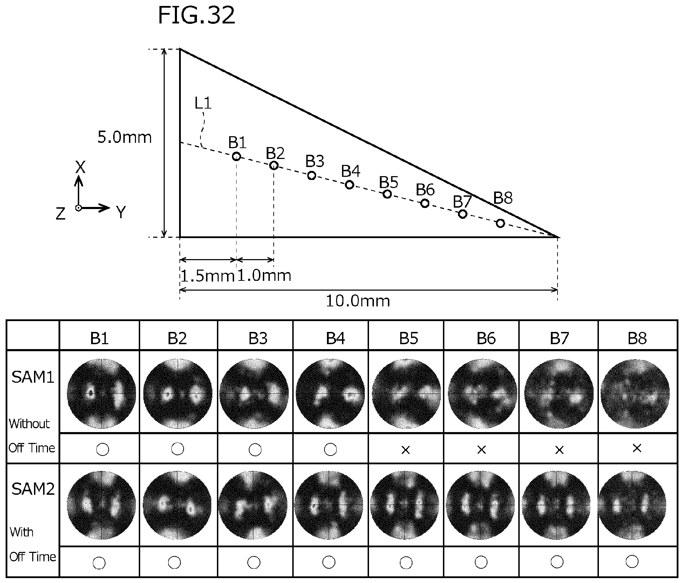

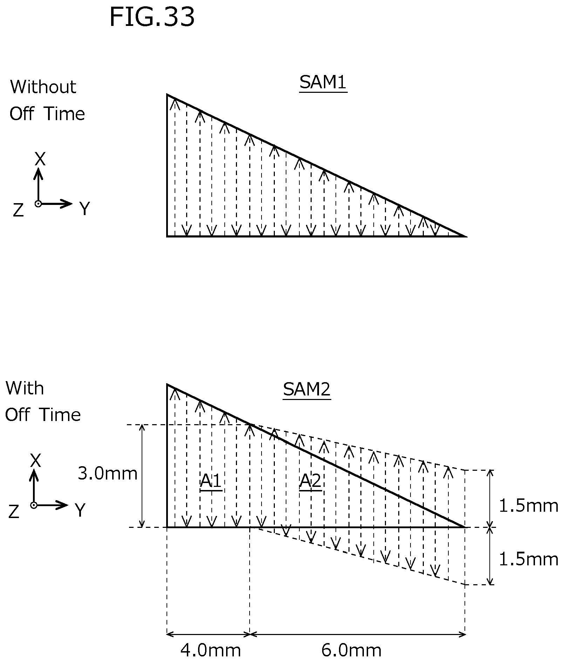

FIG. 32 is a pole figure for illustrating an effect obtained by providing an off time period. FIG. 32 was obtained by the following method. As illustrated in FIG. 33, inorganic structures (each of an amount corresponding to one solidified layer SL1) SAM1 and SAM2 were manufactured which each had a right-angled triangular base with sides of 5 mm in the X direction and 10 mm in the Y direction, and which each had a height of 3 mm in the Z direction. The material of the inorganic structures SAM1 and SAM2 was a Ti-15Mo-5Zr-3Al alloy, specifically, a composition containing, in percent by mass, Mo in an amount of 15%, Zr in an amount of 5%, and Al in an amount of 3%, with the balance being Ti and impurities.

In the case of the inorganic structure SAM1 that is indicated by "Without Off Time", a scanning path was adopted that included reciprocating scanning lines in the X direction. In the melting step, the beam irradiation position was moved in the X direction on one scanning line and the beam was irradiated. Further, after irradiation of the entire length of one scanning line was completed, the beam irradiation position was shifted in the Y direction and the beam was irradiated onto the adjacent scanning line. In the case indicated by "Without Off Time", an off time period was not set. That is, the scanning path was set so that the scanning lines did not go outside the frame of the triangular region.

In the case of the inorganic structure SAM2 that is indicated by "With Off Time" in FIG. 33, an off time period was set. Specifically, in the triangular region, for an area A1 from 0 mm to 4 mm in length in the Y direction, scanning lines were set to the range within the frame of the triangular region. Note that, in the area A1, a single-crystal-like structure was formed.

On the other hand, in an area A2 that was the area other than the area A1, scanning lines were set so that the entire length of each scanning line was 3 mm. In addition, each of the two end points of the respective scanning lines was placed outside the frame of the triangular region. Furthermore, the arrangement of each scanning line was determined so that the length of the portion of the scanning line outside the frame above the triangular region was the same as the length of the portion of the scanning line outside the frame below the triangular region.

During a period in which the beam irradiation position was moving through the portions where the respective scanning lines and the triangular region overlapped, the beam was irradiated onto the beam irradiation position. Further, during a period in which the beam irradiation position was moving through portions where the respective scanning lines and the triangular region did not overlap, irradiation of the beam was stopped to thereby provide an off time period. Note that, the beam conditions other than the scanning path were set to the same conditions for the inorganic structures SAM1 and SAM2 in FIG. 33. Through the above process, the inorganic structures SAM1 and SAM2 in which the <110> crystallographic orientation was preferentially oriented in the build direction Z were manufactured.

In each of the manufactured inorganic structures SAM1 and SAM2, {100} pole figures were determined at observation points B1 to B8 illustrated in FIG. 32. In FIG. 32, a dashed line L1 is a line segment connecting the center position of the side in the X direction of the triangular region and the vertex of the angle that is opposite the side in the X direction. The pitch in the Y direction between the adjacent observation points among the observation points B1 to B8 was 1.0 mm. The obtained pole figures are shown in FIG. 32.

Referring to FIG. 32, the upper row shows pole figures of the inorganic structure SAM1, and the lower row shows pole figures of the inorganic structure SAM2. In the inorganic structure SAM1 for which no off time period was set, the scanning lines became shorter as they progressed in the Y direction. Therefore, a clear pole could not be confirmed from the observation point B5 onward, and the crystallographic orientation confirmed at the observation points B1 to B4 was not inherited by the observation points B5 to B6. On the other hand, in the inorganic structure SAM2 for which an off time period was set, a clear pole was confirmed even from the observation point B5 onward, and all of the observation points B1 to B8 of the inorganic structure SAM2 were a single crystal-like structure.

As described above, by providing an off time period, the preferential crystallographic orientation can be ensured.

Embodiments of the present disclosure have been described above. However, the embodiments described above are merely examples for carrying out the present disclosure. Therefore, the present disclosure is not limited to the above-described embodiments, and can be implemented by appropriately modifying the above-described embodiments within a range not departing from the gist thereof.

### REFERENCE SIGNS LIST

- 1: Inorganic Structure
- CA, CA1, CA2, CA3: Cell Area
- CO1, CO2: Crystallographic Orientation
- SA, SA1, SA2, SA3: Solidified Portion
- SSA: Sub-Solidified Area

## Claims

1. An inorganic structure, comprising:
a plurality of solidified portions composed of an inorganic material,
wherein a plurality of the solidified portions include:
a first solidified portion that has a crystallographic orientation in which a first crystallographic direction is preferentially oriented in a predetermined direction, and
a second solidified portion that has a crystallographic orientation in which a second crystallographic direction that is a different direction from the first crystallographic direction is preferentially oriented in the predetermined direction.

2. The inorganic structure according to claim 1, wherein:
the solidified portion includes a plurality of sub-solidified areas that are each surrounded by a plurality of molten pool interfaces; and
the sub-solidified area includes:
a plurality of cells, and
a plurality of cell interfaces which are interfaces between the cells that are adjacent to each other.

3. The inorganic structure according to claim 1, wherein:
the first solidified portion has a different shape from the second solidified portion.

4. The inorganic structure according to claim 2, wherein:
the sub-solidified area includes:
a plurality of cell areas that are each composed of a plurality of the cells, and
a lamellar interface that is an interface between the cell areas that are adjacent;
and at the lamellar interface, one of the cell areas that are adjacent to each other has a different preferential crystallographic orientation from a crystallographic orientation of the other of the cell areas.

5. The inorganic structure according to claim 2, wherein:
the sub-solidified area includes:
a plurality of cell areas that are each composed of a plurality of the cells, and
an association interface that is an interface between the cell areas that are adjacent;
and at the association interface, one of the cell areas that are adjacent to each other has the same preferential crystallographic orientation as a crystallographic orientation of the other of the cell areas.

6. The inorganic structure according to claim 1, further comprising:
a sintered portion that is a sintered compact of an inorganic material.

7. The inorganic structure according to claim 1, wherein:
a space is formed between the solidified portions that are adjacent.

8. The inorganic structure according to claim 1, wherein:
in the first solidified portion, any one of <001>, <011>, and <111> is preferentially oriented in a build direction ; and
in the second solidified portion, any one of <001>, <011>, and <111> which is different from the crystallographic orientation of the first solidified portion is preferentially oriented in a build direction.

9. The inorganic structure according to claim 1, wherein:
the solidified portion is composed of a single crystal, a single-crystal-like structure, or a polycrystal.

10. A method for manufacturing an inorganic structure according to claim 1, comprising:
a design step of determining a scanning method for scanning a beam for forming the inorganic structure to be manufactured, and
a formation step of scanning the beam based on a scanning method for scanning the beam determined in the design step and melting inorganic powder particles that serve as a raw material of the inorganic structure to form the inorganic structure,
wherein:
the design step includes:
a step of dividing the inorganic structure to be manufactured into a plurality of solidified layers in a build direction,
a step of partitioning the respective solidified layers into a plurality of solidified areas as viewed from the build direction,
an orientation determination step of determining a crystallographic orientation of the respective solidified areas based on mechanical properties required for the respective solidified areas, and
a scanning method determination step of determining a scanning method for scanning a beam in the respective solidified areas based on a determined crystallographic orientation;
and in the formation step:
a layer formation step of supplying the inorganic powder particles onto a base and forming an inorganic powder layer, and
a melting step of scanning the beam based on a scanning method for scanning a beam determined in the scanning method determination step to melt the inorganic powder layer and form the solidified layer that is composed of a plurality of the solidified areas that have the crystallographic orientation determined in the orientation determination step
are repeatedly performed alternately to build a plurality of the solidified layers to form the inorganic structure according to claim 1.

11. The method for manufacturing an inorganic structure according to claim 10, wherein:
in the orientation determination step:
a crystallographic orientation in the build direction of the respective solidified areas is determined as being any one crystallographic direction among <001>, <011>, and <111>; and
in the scanning method determination step:
in the solidified area for which a <001> crystallographic orientation is determined, a scanning direction of the beam when forming the solidified layer of a j-th layer (j is a natural number) is adjusted so as to be orthogonal to a scanning direction of the beam when forming the solidified layer of a j-1th layer that is a layer underneath the j-th layer;
in the solidified area for which a <011> crystallographic orientation is determined, a scanning direction of the beam when forming the solidified layer of a j-th layer is adjusted so as to be parallel to a scanning direction of the beam when forming the solidified layer of the j-1th layer; and
in the solidified area for which a <111> crystallographic orientation is determined, a scanning direction of the beam when forming the solidified layer of a j-th layer is adjusted so as to intersect at 120° with respect to a scanning direction of the beam when forming the solidified layer of the j-1th layer.

12. The method for manufacturing an inorganic structure according to claim 10, wherein:
in the scanning method determination step:
a scanning method for scanning the beam includes information relating to a scanning path of the beam in the solidified area of a size that serves as a standard,
and in the melting step:
in a case where the solidified area to be melted is smaller than the solidified area of the size that serves as a standard, on a scanning path of the beam, the beam is irradiated on a portion that overlaps with the solidified area to be melted, and on the scanning path of the beam, irradiation of a beam is stopped at a portion which does not overlap with the solidified area to be melted.
